# EUROPEAN PATENT SPECIFICATION

(11) **EP 1 782 792 B1**
(45) Date of publication and mention of the grant of the patent: **09.01.2013**
(21) Application number: 06123632.9
(22) Date of filing: 07.11.2006
(51) Int. Cl.: A61K 8/02, A61K 8/49, A61K 8/25, A61Q 1/12, A61Q 17/04

(54) **Ultraviolet ray absorbing composite powder**
Ultraviolette Strahlung absorbierende Verbundpulver
Poudre composite absorbant les rayons ultra-violet

(30) Priority: 07.11.2005 JP 2005322355
(43) Date of publication of application: 09.05.2007
(73) Proprietor: Iwase Cosfa Co., Ltd., Osaka-shi, Osaka-fu (JP)
(72) Inventor: Satonaka, Kenya, Chuo-ku Osaka-fu, Osaka (JP); Yoshioka, Takatsugu, Chuo-ku Osaka-fu, Osaka (JP); Motoki, Takashi, Chuo-ku Osaka-fu, Osaka (JP); Yamagata, Tomohiro, Chuo-ku Osaka-fu, Osaka (JP)
(74) Representative: Eisenführ, Speiser & Partner

(56) References cited:
- EP-A1- 0 545 347
- JP-A- 6 157 245
- US-A- 5 512 094
- US-A- 5 846 310
- MUELLER, S.; HERZOG, B.; GIESINGER, J.; QUASS, K.; OSTERWALDER, U.: "Micronization as a tool in the development of innovative UV filters" SÖFW JOURNAL ; ISSN- 0942-7694, vol. 131, no. 7, July 2005 (2005-07), pages 32-39, XP002418657

## Description

### FIELD OF THE INVENTION

The instant application relates to a composite powder having core particle and benzotriazol UV absorber coating which is coated on the surface of the core particle, and cosmetic composition comprising the same.

### ART RELATED

Ultraviolet light is divided into UV-C (200-280nm wavelength) which is absorbed by the ozone layer, UV-B (280-320nm) which causes sunburn, and UV-A (320-400nm) which promotes suntan after exposure. According to recent studies, UV-A and UV-B cause harmful effects including not only sunburn and suntan but also pigment deposition, dry skin, skin roughness and skin aging. In addition, depletion of the ozone layer results in increased UV radiation reaching the Earth's surface, which in turn can lead to a greater chance of overexposure to UV radiation and the related effects to the skin. There are increasing desire for cosmetics with UV-protecting property.

Organic ultraviolet absorbers, which have been used for protecting against UV radiation, have various problems in using the same for manufacturing cosmetic products, for examples, (1) being less soluble to oils, especially silicone oils, which are conventionally used in cosmetics; (2) usually cause sticky feelings to the skin; (3) being less stable against light; (4) discoloration and/or unpleasant odor occurs upon time. On the other hand, conventionally used inorganic UV absorbers have problems such as (1) cause squeaky feeling, (2) result in white appearance when applied to the skin and (3) act as light catalyst. Due to those problems, the amount of the inorganic UV absorbers being added to cosmetic product has been limited. In addition, many conventional cosmetic products with UV protecting property comprises sole UV absorber and there are no products having UV protecting properties against wide range of UV rays including UV-A and UV-B.

A new UV absorber which is excellent in light stability and can protect against wide range UV rays, 2,2'-methylenebis[6-(2H-benzotriazolyl-2-yl)-4-(1,1,3,3-tetramethylbutyl) phenol] has been developed (see USP 5,225,951). In this specification, the compound is abbreviated as "MBBT". This organic UV absorber is insoluble to water as well as to oils used in cosmetic products and provides the UV protecting effect by absorbing, scattering and reflecting UV rays. The UV protecting effect based on the UV ray scattering and reflecting may vary depending on the particle size of the compound and particle size range of 150-200nm is optimal. In addition, combined use of MBBT with another UV absorber will synergistically increase the UV protecting property of the compound.

USP 6,235,271 discloses a product comprising micronized MBBT of the optimal particle size dispersed in water. Said aqueous dispersion of micronized MBBT is used conveniently for manufacturing cosmetic product and has been available on market (TINOSORB® M, Ciba Specialty Chemicals, Basel, Switzerland). However, said product cannot be used for manufacturing non-aqueous formulation. In addition, MBBT particles will aggregate when a salt is added to the dispersion and the UV protecting action of the aggregated MBBT will decrease significantly. For manufacturing water in oil type emulsion, salts such as sodium sulfate and sodium chloride are added to stabilize the emulsion and therefore, it was difficult to manufacture water in oil emulsion with the aqueous dispersion of MBBT. In addition, a large amount of MBBT in a cosmetic composition may cause unpleasant texture of the product.

Japanese Patent Application Laid Open No. 2004-168913 proposes incorporating MBBT particles in a polymer matrix which is soluble in organic solvents. However, the MBBT particles tend to aggregate upon incorporating the same into the polymer matrix and therefore, hardly maintain the optimal particle size. Accordingly, the resulting product could not provide enough UV-protecting effect.

### SUMMARY OF THE INVENTION

An object of the application is to provide a transparent composite powder which has excellent ultraviolet-absorbing ability and is comfortable to wear. A further object of the application is to provide a cosmetic composition having a high ultraviolet-protecting property comprising said composite powder.

The instant application provides a composite powder comprising a plurality of composite powder particles, wherein (each of) the composite powder particle consists of an inorganic or organic core particle having an average particle diameter of 0.5-100µm, and a compound of formula (1): 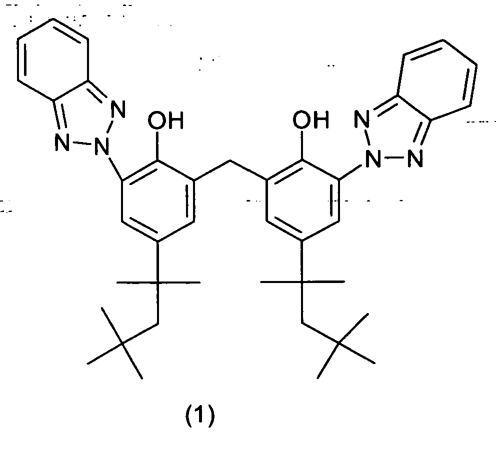 which is coated on the surface of the core particle.
The benzotriazol type ultraviolet-absorber of formula (1) is insoluble in water or oils used for manufacturing cosmetic compositions obtainable by
mixing the core particles and the compound of formula (1) in an aqueous solvent under the presence of an aqueous soluble salt of a metal selected from the group consisting of Al, Mg, Ca, Zn, Zr and Ti, whereby the compound of formula (1) adsorbs to the surface of the core particles,
wherein the amount of the aqueous soluble salt of a metal is 0.25 - 2 parts by weight per one part by weight of the compound of formula (1).

Further, the instant application provides a cosmetic composition comprising the above described composite powder. Preferably, the cosmetic composition further comprises an additional UV absorber selected from the group consisting of a methoxy cinnamate derivative such as octyl methoxy cinnamate, isoamyl methoxy cinnamate or butyl methoxydibenzoylmethane (BMDBM). In particular the cosmetic composition comprises one or more additional UV-absorbers s elected from the group consisting of (i) methoxy cinnamate derivatives, such as octyl methoxy cinnamate, isoamyl methoxy cinnamate and mixtures thereof, (ii) butyl methoxydibenzoylmethane (BMDBM) and (iii) mixtures thereof. Octyl methoxy cinnamate is particulary preferred as additional UV-absorber according to the invention. By the combined use of the composite powder of the invention and a methoxy cinnamate derivative or BMDBM, the photostability of the methoxy cinnamate derivative or BMDBM can be enhanced and the ultraviolet protecting property of the cosmetic composition will be synergistically increased.

Further more, the instant application provides a method for manufacturing the composite powder of the invention, which comprises the step of mixing the core particles and the compound of formula (1) in an aqueous solvent under the presence of an aqueous soluble salt of a metal selected from the group consisting of Al, Mg, Ca, Zn, Zr and Ti whereby the compound of formula (1) adsorbs to the surface of the core particles wherein the amount of the aqueous soluble salt of a metal is 0.25 - 2 parts by weight per one part by weight of the compound of formula (1).
Furthermore, the instant application relates to the use of a compound of formula (1), as described hereinbefore, in the manufacture of a composite powder according to the present invention.
Furthermore, the instant application relates to the use of a compound of formula (1), as described hereinbefore, in the manufacture of a cosmetic composition according to the present invention.
Furthermore, the instant application relates to the use of an inorganic or organic (core) particle having an average particle diameter of 0.5-100µm as a means for adsorbing a compound of formula (1) as described hereinbefore. In particular this use relates to the manufacture of a composite powder according to the present invention, characterized in that the compound of formula (1) as described hereinbefore is adsorbed to the surface of the core particle.

### BRIEF DESCRIPTION OF DRAWINGS

Figure 1 depicts the graph showing the result of test example 1.

Figure 2 is an electron micrograph of the composite powder obtained in example 1.

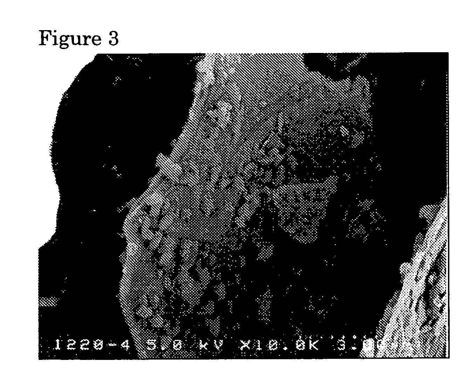

Figure 3 is an electron micrograph of the composite powder obtained in Example 5.

### BEST MODE FOR CARRYING OUT THE INVENTION

The compound of formula (1), a benzotriazole type UV absorber, is preferably in the form of fine particles having an average particle diameter of equal to or less than 200nm, more preferably, 150-200 nm. Higher UV protecting property of the resulting composite powder can be achieved by employing the particles within said size range. The compound of formula (1) is available on market and TINOSORB® M (Ciba Specialty Chemicals, Basel, Switzerland) can preferably used in this invention. TINOSORB® M is an aqueous dispersion containing 50% of powdery compound of formula (1) having an average particle diameter of 200nm or less.

The inorganic or organic core particle having an average particle diameter of 0.5-100µm may be any material used for manufacturing cosmetic products. For example, inorganic particles such as talc, kaolin, sericite, white mica, bronze mica, black mica, synthetic mica, red mica, lithia mica, vermiculite, magnesium carbonate, calcium carbonate, diatomite, magnesium silicate, calcium silicate, aluminum silicate, barium silicate, barium sulfate, strontium silicate, metal tungstate, silica, hydroxyapatite, zeolite, boron nitride and ceramics powder, organic particles such as nylon powder, silicone powder, polyethylene powder, polystyrene powder, benzoguanamine powder, polyethylene tetrafluoride, distyrene benzene polymer powder, epoxy powder, acrylic powder, microcrystalline cellulose and urethane powder, as well as white pigments such as titanium oxide, micro titanium dioxide, zinc oxide and micro zinc oxide. Among them, talc, mica, sericite, kaolin and barium sulfate are preferably used. Lamellar particles are especially useful since the resulting composite particles will have uneven surface which provide less supecular reflection and cause less glossy-finish. When spherical particles such as silica or cellulose are used as core particles, the surface of the resulting composite particle becomes uneven due to the treatment with the compound of formula (1) and therefore, the composite powder having a higher diffuse reflection property can be obtained and the cosmetic composition comprising said composite powder can provide an excellent power to minimize the appearance of skin pores.

The average particle diameter of the core particles is preferably 0.5-100µm, more preferably, 1-50µm. When the average particle size is less than 0.5µm, the composite powder with the good properties cannot be obtained using the benzotriazole UV-absorber having an average particle size of 200nm or less. In contrast, when the particle diameter of the core particles is 100µm or more, the texture of the resulting composite powder becomes unpleasant.

The composite powder of the invention may be manufactured by mixing the core particles and the compound of formula (1) in an aqueous solvent under the presence of an aqueous soluble salt of a metal selected from the group consisting of Al, Mg, Ca, Zn, Zr and Ti whereby coating the core particle by the compound of formula (1). According to the method of the invention, the particles of the compound of formula (1) are adsorbed to the surface of the core particles in an oriented manner. Examples of the aqueous soluble salt of a metal selected from the group consisting of Al, Mg, Ca, Zn, Zr and Ti used in the instant method may include aluminum sulfate, aluminum chloride, aluminum nitrate, aluminum calcium sulfate, magnesium chloride, magnesium sulfate, magnesium nitrate, calcium sulfate, calcium chloride, calcium nitrate, calcium acetate, zinc chloride, zinc nitrate, zinc sulfate, zinc acetate, zirconium sulfate, zirconium chloride, titanium oxysulfate and titanium tetrachloride. Among the above, aluminum sulfate is especially useful in view of its availability. The metal salts may be used as aqueous solutions thereof.

Upon manufacturing the composite powder of the present invention, the amount of the benzotriazole type UV-absorber of formula (1) may be 1 wt% or more, and 35wt% or less, especially, 1-35 wt%, more especially 3-30 wt% based on the total amount of the core particles and the UV absorber. By adjusting the amount of the UV-absorber, the resulting composite powder can exhibit good UV protecting effect as well as good and comfortable texture.

According to the method for manufacturing the composite powder of the invention, the amount of the salt of a metal selected from the group consisting of Al, Mg, Ca, Zn, Zr and Ti per one part by weight of the compound of formula (1) may preferably be 1/4 (0,25) part by weight or more so that the particles of the benzotriazole type UV-absorber are adsorbed well to the surface of the core particles, and 2 parts by weight or less in view of the comfortable texture of the resulting composite powder, and especially, 1/4-2, and more especially, 1/2-1 parts by weight.

The composite powder of the invention consists of plurality of composite powder particles that comprises core particles and compound of formula (1) adsorbed thereto in an oriented manner. The details of the surface treatment is not specifically limited and may be carried out based on known procedures. For example, the core particles are dispersed in water and 1-35 wt% of the compound of formula (1), based on the total amount of the core particles and the compound of formula (1), is added to the dispersion and stirred vigorously. The oriented adsorption of a part of the benzotriazole compound to the surface of the core particles occurs during this stage. Then, an aqueous soluble salt of a metal selected from the group consisting of Al, Mg, Ca, Zn, Zr and Ti in an amount of 1/4-2 parts by weight per one part by weight of the compound of formula (1) in the form of aqueous solution is added to the mixture. By adding the aqueous solution of the salt, the oriented adsorption of the particles of the compound of formula (1) to the surface of the core particles is completed. Thus obtained mixture is then dehydrated and washed by centrifugation or the like, and dried at 80-120°C to give the desired composite powder having UV ray protecting property.

Thus obtained composite powder of the invention has the structure wherein particles of the insoluble benzotriazole UV absorber of formula (1) are adsorbed uniformly to the surface of the core particle and the UV absorber particles are not aggregated each other. Due to the structure, the composite particle behaves as if it is primary particle consisting of single particle. Accordingly, the composite powder of the invention comprising plurality of the composite particles is highly transparent, has smooth and comfortable texture, and provides an excellent UV ray protecting property. The composite powder of the invention is preferably used for manufacturing cosmetic products.

The composite powder of the invention may be used without further processing or the surface of the composite particle may include treated with a hydrophobic agent to give hydrophobic powder. Examples of hydrophobic agents for the hydrophobic treatment may be silicone agent, fatty acid, fatty acid soap and fatty acid ester. Examples of silicone agents may include silicone oils such as methylhydrogenpoly siloxane, dimethylpoly siloxane and methylphenylpoly siloxane, alkyl silanes such as methyltrimethoxysilane, ethyltrimethoxysilane, hexyl-trimethoxysilane and octyltrimethoxysilane, fluoroalkyl silanes such as trifluoro-methylethyltrimethoxysilane and heptadecafluorodecyl trimethoxysilane. Examples of fatty acids may include palmitic acid, isostearic acid, stearic acid, lauric acid, myristic acid, behenic acid, oleic acid, rosin acid and 1,2-hydroxystearic acid. Examples of fatty acid soaps may include aluminum stearate, calcium stearate and aluminum 1,2-hydroxystearate. Examples of fatty acid esters may include dextrin fatty acid ester, cholesterol fatty acid ester, sucrose fatty acid ester and starch fatty acid ester. The hydrophobic treatment of the composite powder of the invention may be carried out with one or more of the hydrophobic agents as above and according to a conventionally known method.

The instant invention further provides a cosmetic composition comprising the composite powder of the invention. The cosmetic composition of the present invention may comprise further ingredients which are added to conventional cosmetic products, if desired. Examples of the ingredients other than the composite powder of the invention may include solid/half solid oil such as vaseline, lanoline, ceresin, microcrystalline wax, carnauba wax, candelilla wax, higher fatty acid and higher alcohol, fluid oil such as squalan, fluid paraffin, ester oil, diglyceride, triglyceride, silicone oil, olive oil, avocado oil and mink oil, fluoride oils such as perfluoro polyether, perfluorodecalin and perfluorooctane, water- and oil-soluble polymer, surface active agent, inorganic and organic pigments, silicone, metal soap, lecithin, amino acid, collagen, polymer, inorganic or organic pigment whose surface is treated by fluoride compound, colorant such as tar color and natural color, ethanol, antiseptic agent, antioxidant, dye, thickener, pH adjustor, flavoring agent, UV absorber, moisturizing agent, blood circulation improver, cool feeling agent, bactericide, skin activating agent and water. Those ingredients may be added to the cosmetic composition of the invention as long as they deteriorate the object of the invention.

The cosmetic composition of the present invention may preferably comprise an additional UV absorbing agent selected from a methoxy cinnamate derivative such as octyl methoxy cinnamate or isoamyl methoxy cinnamate and butyl methoxydibenzoylmethane (BMDBM). By the combined use of the composite powder and a methoxy cinnamate derivative or BMDBM, a synergistic UV-protecting property can be provided to the resulting composition. Preferably, the additional UV absorbing agent is a methoxy cinnamate derivative, especially, octyl methoxy cinnamate.

The octyl methoxy cinnamate used in the instant invention is a transparent pale yellow liquid with weak characteristic odor and is a UV-B absorber widely used for manufacturing cosmetic products. The compound is commercially available, for example PARSOL® MCX (DSM Nutritional Products), Uvinul® MC-80 (BASF), NOMUCORT® TAB (Nisshin Oillio) and Escarole^{®} (ISP). Isoamyl methoxy cinnamate is also available on market as Neo Heliopan^{®} E1000 (H&R).

The cosmetic composition of the invention may further comprise one or more additional UV filter substances and adjuvants shown in the table below:

| Suitable UV filter substances and adjuvants which can be additionally used with the UV Ray absorbing composite powder according to the present invention | | |
|---|---|---|
| No. | Chemical Name | CAS No. |
| 1 | (+/-)-1,7,7-trimethyl-3-[(4-methylphenyl)methylene]bicyclo-[2.2.1]heptan-2-one; p-methyl benzylidene camphor | 36861-47-9 |
| 2 | 1,7,7-trimethyl-3-(phenylmethylene)bicyclo[2.2.1]heptan-2-one; benzylidene camphor | 15087-24-8 |
| 3 | (2-Hydroxy-4-methoxyphenyl)(4-methylphenyl)methanone | 1641-17-4 |
| 4 | 2,4-dihydroxybenzophenone | 131-56-6 |
| 5 | 2,2',4,4'-tetrahydroxybenzophenone | 131-55-5 |
| 6 | 2-Hydroxy-4-methoxy benzophenone; | 131-57-7 |
| 7 | 2-Hydroxy-4-methoxy benzophenone-5-sulfonic acid | 4065-45-6 |
| 8 | 2,2'-dihydroxy-4,4'-dimethoxybenzophenone | 131-54-4 |
| 9 | 2,2'-Dihydroxy-4-methoxybenzophenone | 131-53-3 |
| 10 | Alpha-(2-oxoborn-3-ylidene)toluene-4-sulphonic acid and its salts (Mexoryl SL) | 56039-58-8 |
| 11 | 1-[4-(1,1-di methylethyl)phenyl]-3-(4-methoxyphenyl)propane-1,3-dione (Avobenzone) | 70356-09-1 |
| 12 | Methyl N,N,N-trimethyl-4-[(4,7,7-trimethyl-3-oxobicyclo[2,2,1]hept-2-ylidene)methyl]anilinium sulphate (Mexoryl SO) | 52793-97-2 |
| 22 | 3,3,5-Trimethyl cyclohexyl-2-hydroxy benzoate; homosalate | 118-56-9 |
| 23 | Isopentyl p-methoxycinnamate; isoamyl methoxy cinnamate | 71617-10-2 |
| 27 | Menthyl-o-aminobenzoate | 134-09-8 |
| 28 | Menthyl salicylate | 89-46-3 |
| 29 | 2-Ethylhexyl 2-cyano,3,3-diphenylacrylate; octocrylene | 6197-30-4 |
| 30 | 2- ethylhexyl 4- (dimethylamino)benzoate | 21245-02-3 |
| 31 | 2- ethylhexyl 4- methoxycinnamate; octyl methoxy cinnamate | 5466-77-3 |
| 32 | 2- ethylhexyl salicylate | 118-60-5 |
| 33 | Benzoic acid,4,4',4"-(1,3,5-triazine-2,4,6-triyltriimino)tris-,tris(2-ethylhexyl)ester; 2,4,6-Trianilino-(p-carbo-2'-ethylhexyl-1'-oxi)-1,3,5-triazine (Octyl Triazone) | 88122-99-0 |
| 34 | 4- aminobenzoic acid | 150-13-0 |
| 35 | Benzoic acid, 4-amino-, ethyl ester, polymer with oxirane | 113010-52-9 |
| 38 | 2- phenyl- 1 H- benzimidazole- 5- sulphonic acid; phenylbenzimidazolsulfonic acid | 27503-81-7 |
| 39 | 2-Propenamide, N-[[4-[(4,7,7-trimethyl-3-oxobicyclo[2.2.1]hept-2-ylidene)methyl]phenyl]methyl]-, homopolymer | 147897-12-9 |
| 40 | Triethanolamine salicylate | 2174-16-5 |
| 41 | 3, 3'-(1,4-phenylenedimethylene)bis[7, 7-dimethyl- 2-oxo-bicyclo[2.2.1]heptane-1 methanesulfonic acid] (Cibafast H) | 90457-82-2 |
| 42 | Titanium dioxide | 13463-67-7 |
| 44 | Zinc oxide | 1314-13-2 |
| 45 | 2,2'-Methylene-bis-[6-(2H-benzotriazol-2-yl)-4-(1,1,3,3-tetramethylbutyl)-phenol] (Tinosorb M) | 103597-45-1 |
| 46 | 2,4-bis{[4-(2-ethylhexyloxy)-2-hydroxy]-phenyl}-6-(4-methoxyphenyl)-(1,3,5)-triazine (Tinosorb S) | 187393-00-6 |
| 47 | 1H-Benzimidazole-4,6-disulfonic acid, 2,2'-(1,4-phenylene)bis-, disodium salt | 180898-37-7 |
| 48 | Benzoic acid, 4,4'-[[6-[[4-[[(1,1-dimethylethyl)amino]-carbonyl]phenyl]amino]1,3,5-triazine-2,4-diyl]diimino]bis-, bis(2-ethylhexyl)ester; diethylhexyl butamido triazone (Uva-sorb HEB) | 154702-15-5 |
| 49 | Phenol, 2-(2H-benzotriazol-2-yl)-4-methyl-6-[2-methyl-3-[1,3,3,3-tetramethyl-1-[(trimethylsilyl)oxy]disiloxanyl]propyl]-; drometrizole trisiloxane (Mexoryl XL) | 155633-54-8 |
| 50 | Dimethicodiethylbenzalmalonate; Polysilicone 15 (Parsol SLX) | 207574-74-1 |
| 51 | Benzenesulfonic acid, 3-(2H-benzotriazol-2-yl)-4-hydroxy-5-(1-methylpropyl)-, monosodium salt (Tinogard HS) | 92484-48-5 |
| 52 | Benzoic acid, 2-[4-(diethylamino)-2-hydroxybenzoyl]-, hexyl ester (Uvinul A Plus) | 302776-68-7 |
| 53 | 1-Dodecanaminium, N-[3-[[4-(dimethylamino)benzoyl]amino]propyl]N,N-dimethyl-, salt with 4-methylbenzenesulfonic acid (1:1) (Escalol HP610) | 156679-41-3 |
| 54 | 1-Propanaminium, N,N,N-trimethyl-3-[(1-oxo-3-phenyl-2-propenyl)amino]-, chloride | 177190-98-6 |
| 55 | 1 H-Benzimidazole-4,6-disulfonic acid, 2,2'-(1,4-phenylene)bis- | 170864-82-1 |
| 56 | 1,3,5-Triazine, 2,4,6-tris(4-methoxyphenyl)- | 7753-12-0 |
| 57 | 1,3,5-Triazine, 2,4,6-tris[4-[(2-ethylhexyl)oxy]phenyl]- | 208114-14-1 |
| 58 | 1-Propanaminium, 3-[[3-[3-(2H-benzotriazol-2-yl)-5-(1,1-dimethylethyl)-4-hydroxyphenyl]-1-oxopropyl]amino]-N,N-diethyl-N-methyl-, methyl sulfate (salt) | 340964-15-0 |
| 59 | 2-Propenoic acid, 3-(1 H-imidazol-4-yl)- | 104-98-3 |
| 60 | Benzoic acid, 2-hydroxy-, [4-(1-methylethyl)phenyl]methyl ester | 94134-93-7 |
| 61 | 1,2,3-Propanetriol, 1-(4-aminobenzoate) (Glyceryl PABA) | 136-44-7 |
| 62 | Benzeneacetic acid, 3,4-dimethoxy-a-oxo- | 4732-70-1 |
| 63 | 2-Propenoic acid, 2-cyano-3,3-diphenyl-, ethyl ester | 5232-99-5 |
| 64 | Anthralinic acid, p-menth-3-yl ester | 134-09-8 |
| 65 | 2,2'-bis(1,4-phenylene)-1H-benzimidazole-4,6-disulphonic acid mono sodium salt or Disodium phenyl dibenzimidazole tetrasulfonate (Neo Heliopan AP) | 349580-12-7, |
| 66 | 1,3,5-Triazine-2,4,6-triamine, N,N'-bis[4-[5-(1,1-dimethylpropyl)-2-benzoxazolyl]phenyl]-N"-(2-ethylhexyl)-(Uvasorb K2A) | 288254-16-0 |
| 67 | 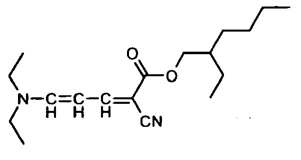 | |
| 68 | sterols (cholesterol, lanosterol, phytosterols), as described in WO034 1675 | |
| 69 | mycosporines and/or mycosporine-like amino acids as described in WO2002039974, e.g. Helioguard 365 from Milbelle AG, isolated mycosporine like amino acids from the red alga porphyra umbilicalis (INCI: Porphyra Umbilicalis) that are encapsulated into liposomes,) | |
| 70 | alpha-lipoic-acid as described in DE 10229995 | |
| 71 | synthetic organic polymers as described in EP 1371358, [0033]-[0041] | |
| 72 | phyllosilicates as described in EP 1371357 [0034]-[0037] | |
| 73 | silica compounds as described in EP1371356, [0033]-[0041] | |
| 74 | inorganic particles as described in DE10138496 [0043]-[0055] | |
| 75 | latex particles as described in DE10138496 [0027]-[0040] | |
| 76 | 1H-Benzimidazole-4,6-disulfonic acid, 2,2'-(1,4-phenylene)bis-, disodium salt ; Bisimidazylate (Neo Heliopan APC) | 180898-37-7 |
| 77 | 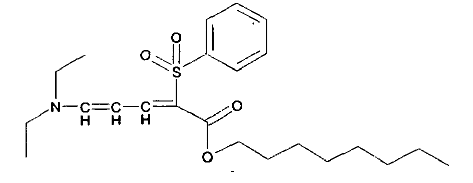 | |
| 78 | 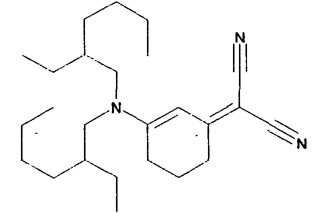 | |
| 79 | 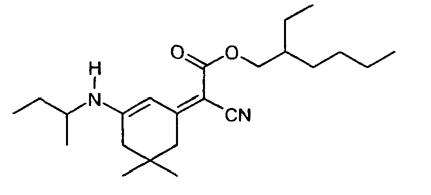 E or Z isomer or mixture of E/Z isomers | |
| 80 | 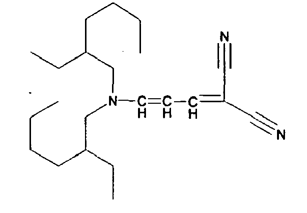 | |
| 81 | Di-2-ethylhexyl-3,5-dimethoxy-4-hydroxy-benzalmalonate (Oxynex ST, EMD Chemicals, as described in US 20040247536) | |
| 82 | 2,4,6-Tris-1,1',4',1"-terphenyl-4-yl-1,3,5-triazine | |

The form or product type of the instant cosmetic composition is not specifically limited and may be in the form of make up products such as powdery foundation, cream foundation, oil base foundation, two-way foundation, stick type foundation, loose powder, eye shadow, cheek blush and lipstick, skin care products such as lotion, emulsion and cream as well as body cosmetics such as body powder and baby powder, further in particular as liquid foundation, pressed powder, W/O emulsion foundation, sunscreen cream and makeup base.
Preferably the cosmetic composition according to the invention is selected from the group consisting of powdery foundation, liquid foundation, loose powder, pressed powder, stick foundation, W/O emulsion foundation, cheek blush, sunscreen cream, lipstick and makeup base.

As water- and oil-containing emulsions (e.g. W/O, O/W, O/W/O and W/O/W emulsions or microemulsions) the preparations contain, for example, from 0.1 to 30 % by weight, preferably from 0.1 to 15 % by weight and especially from 0.5 to 10 % by weight, based on the total weight of the composition, of one or more UV absorbers including the composite powder of the invention, from 1 to 60 % by weight, especially from 5 to 50 % by weight and preferably from 10 to 35 % by weight, based on the total weight of the composition, of at least one oil component, from 0 to 30 % by weight, especially from 1 to 30 % by weight und preferably from 4 to 20 % by weight, based on the total weight of the composition, of at least one emulsifier, from 10 to 90 % by weight, especially from 30 to 90 % by weight, based on the total weight of the composition, of water, and from 0 to 88.9 % by weight, especially from 1 to 50 % by weight, of further cosmetically acceptable adjuvants.

The cosmetic or pharmaceutical compositions/preparations according to the invention may also contain one or one more additional compounds as described below.

### Fatty alcohols

Guerbet alcohols based on fatty alcohols having from 6 to 18, preferably from 8 to 10 carbon atoms including cetyl alcohol, stearyl alcohol, cetearyl alcohol, oleyl alcohol, octyldodecanol, benzoate of C12-C15 alcohols, acetylated lanolin alcohol, etc..

### Esters of fatty acids

Esters of linear C6-C24 fatty acids with linear C3-C24 alcohols, esters of branched C6 C13carboxylic acids with linear C6-C24 fatty alcohols, esters of linear C6-C24 fatty acids with branched alcohols, especially 2-ethylhexanol, esters of hydroxycarboxylic acids with linear or branched C6-C22 fatty alcohols, especially dioctyl malates, esters of linear and/or branched fatty acids with polyhydric alcohols (for example propylene glycol, dimer diol or trimer triol) and/or Guerbet alcohols, for example caproic acid, caprylic acid, 2-ethylhexanoic acid, capric acid, lauric acid, isotridecanoic acid, myristic acid, palmitic acid, palmitoleic acid, stearic acid, isostearic acid, oleic acid, elaidic acid, petroselinic acid, linoleic acid, linolenic acid, elaeostearic acid, arachidic acid, gadoleic acid, behenic acid and erucic acid and technical-grade mixtures thereof (obtained, for example, in the pressure removal of natural fats and oils, in the reduction of aldehydes from Roelen's oxosynthesis or in the dimerisation of unsaturated fatty acids) with alcohols, for example, isopropyl alcohol, caproic alcohol, capryl alcohol, 2-ethylhexyl alcohol, capric alcohol, lauryl alcohol, isotridecyl alcohol, myristyl alcohol, cetyl alcohol, palmoleyl alcohol, stearyl alcohol, isostearyl alcohol, oleyl alcohol, elaidyl alcohol, petroselinyl alcohol, linoyl alcohol, linolenyl alcohol, elaeostearyl alcohol, arachidyl alcohol, gadoleyl alcohol, behenyl alcohol, erucyl alcohol and brassidyl alcohol and technical-grade mixtures thereof (obtained, for example, in the high-pressure hydrogenation of technical-grade methyl esters based on fats and oils or aldehydes from Roelen's oxosynthesis and as monomer fractions in the dimerisation of unsaturated fatty alcohols).
Examples of such ester oils are isopropylmyristate, isopropylpalmitate, isopropylstearate, isopropyl isostearate, isopropyloleate, n-butylstearate, n-hexyllaurate, n-decyloleate, isooctylstearate, iso-nonylstearate, isononyl isonon-anoate, 2-ethylhexylpalmitate, 2-hexyllaurate, 2-hexyldecylstearate, 2-octyldodecylpalmitate, oleyloleate, oleylerucate, erucyloleate, erucylerucate, cetearyl octanoate, cetyl palmitate, cetyl stearate, cetyl oleate, cetyl behenate, cetyl acetate, myristyl myristate, myristyl behenate, myristyl oleate, myristyl stearate, myristyl palmitate, myristyl lactate, propylene glycol dicaprylate/caprate, stearyl heptanoate, diisostearyl malate, octyl hydroxystearate, etc..

### Other adjuvants

Alpha glucosylrutin (CAS No. 130603-71-3), 2-butyloctyl o-hydroxybenzoate (CAS No. 190085-41-7), vitamin E (CAS No. 1406-18-4), vitamin E acetate (CAS No. 58-95-7), diethylhexyl 2,6- naphthalate, di-n-butyl adipate, di(2-ethylhexyl)-adipate, di(2-ethylhexyl)-succinate and diisotridecyl acelaat, and also diol esters, such as ethylene glycol dioleate, ethylene glycol diisotride-canoate, propylene glycol di(2-ethylhexanoate), propylene glycol diisostearate, propylene glycol dipelargonate, butanediol diisostearate and neopentyl glycol dicaprylate. Esters of C6-C24 fatty alcohols and/or Guerbet alcohols with aromatic carboxylic acids, saturated and/or unsaturated, especially benzoic acid, esters of C2-C12dicarboxylic acids with linear or branched alcohols having from 1 to 22 carbon atoms or polyols having from 2 to 10 carbon atoms and from 2 to 6 hydroxy groups, or iminodisuccinic acid and imiondisuccinic acid salts [CAS 7408-20-0] or latex particles, aloe vera, chamomile, ginko biloba, ginseng, coenzyme Q10, laminaria ochroleuca extract, magnolia oborata extract, melalenca alternifolia leaf oil, rubus idaeus seed oil, vaccinium macrocarpon seed oil, pumpkin seed extract, pumpkin seed oil, grape seed extract, carnosine, alpha-arbutin, madecassoside, termino-laside, tetrahydrocurcuminoids (THC), mycosporines, mycosporine like amino acids from the red alga porphyra umbilicalis, mycosporine-like amino acids (as described in WO2002039974), cis-9-octadecenedioic acid, lipoic acid,laurimino dipropiomic acid tocopheryl phosphates (LDTP), microcrystalline cellulose (MCC), polycarbonates as described in WO 0341676, sterols (cholesterol, lanosterol, phytosterols), as described in WO0341675 .and linear poly-alpha-glucans as described in US6616935.

### Natural or synthetic triglycerides including glyceryl esters and derivatives

Di- or tri-glycerides, based on C6-C18 fatty acids, modified by reaction with other alcohols (caprylic/capric triglyceride, wheat germ glycerides, etc.). Fatty acid esters of polyglycerin (polyglyceryl-n such as polyglyceryl-4 caprate, polyglyceryl-2 isostearate, etc. or castor oil, hydrogenated vegetable oil, sweet almond oil, wheat germ oil, sesame oil, hydrogenated cottonseed oil, coconut oil, avocado oil, corn oil, hydrogenated castor oil, shea butter, cocoa butter, soybean oil, mink oil, sunflower oil, safflower oil, macadamia nut oil, olive oil, hydrogenated tallow, apricot kernel oil, hazelnut oil, borago oil, etc.
Waxes including esters of long-chain acids and alcohols as well as compounds having wax-like properties, e.g., carnauba wax, beeswax (white or yellow), lanolin wax, candellila wax, ozokerite, japan wax, paraffin wax, microcrystalline wax, ceresin, cetearyl esters wax, synthetic beeswax,etc. Also, hydrophilic waxes as Cetearyl Alcohol or partial glycerides.

### Pearlescent waxes

Alkylene glycol esters, especially ethylene glycol distearate; fatty acid alkanolamides, especially coco fatty acid diethanolamide; partial glycerides, especially stearic acid monoglyceride; esters of polyvalent, unsubstituted or hydroxy-substituted carboxylic acids with fatty alcohols having from 6 to 22 carbon atoms, especially long-chained esters of tartaric acid; fatty substances, for example fatty alcohols, fatty ketones, fatty aldehydes, fatty ethers and fatty carbonates, which in total have at least 24 carbon atoms, especially laurone and distearyl ether; fatty acids, such as stearic acid, hydroxystearic acid or behenic acid, ring-opening products of olefin epoxides having from 12 to 22 carbon atoms with fatty alcohols having from 12 to 22 carbon atoms and/or polyols having from 2 to 15 carbon atoms and from 2 to 10 hydroxy groups, and mixtures thereof.

### Hydrocarbon oils

Mineral oil (light or heavy), petrolatum (yellow or white), microcrystalline wax, paraffinic and isoparaffinic compounds, hydrogenated isoparaffinic molecules as polydecenes and polybutene, hydrogenated polyisobutene, squalane, isohexadecane, isododecane and others from plant and animal kingdom.

### Silicones or siloxanes (organosubstituted polysiloxanes)

Dimethylpolysiloxanes, methylphenylpolysiloxanes, cyclic silicones, and also amino-, fatty acid-, alcohol-, polyether-, epoxy-, fluorine-, glycoside- and/or alkyl-modified silicone compounds, which at room temperature may be in either liquid or resinous form. Linear polysiloxanes, dimethicone (Dow Corning 200 fluid, Rhodia Mirasil DM), dimethiconol, cyclic silicone fluids, cyclopentasiloxanes volatiles (Dow Corning 345 fluid), phenyltrimethicone (Dow Corning 556 fluid). Also suitable are simethicones, which are mixtures of dimethicones having an average chain length of from 200 to 300 dimethylsiloxane units with hydrogenated silicates. A detailed survey by Todd et al. of suitable volatile silicones may in addition be found in Cosm. Toil. 91, 27 (1976).

### Fluorinated or perfluorinated oils

Perfluorhexane, dimethylcyclohexane, ethylcyclopentane, polyper-fluoromethylisopropyl ether.

### Emulsifiers

Any conventionally usable emulsifier can be used for the compositions. Emulsifier systems may comprise for example: carboxylic acids and their salts: alkaline soap of sodium, potassium and ammonium, metallic soap of calcium or magnesium, organic basis soap such as Lauric, palmitic, stearic and oleic acid etc.. Alkyl phosphates or phosphoric acid esters, acid phosphate, diethanolamine phosphate, potassium cetyl phosphate. Ethoxylated carboxylic acids or polyethyleneglycol esters, PEG-n acylates. Linear fatty alcohols having from 8 to 22 carbon atoms, branched from 2 to 30 mol of ethylene oxide and/or from 0 to 5 mol propylene oxide with with fatty acids having from 12 to 22 carbon atoms and with alkylphenols having from 8 to 15 carbon atoms in the alkyl group. Fatty alcohol polyglycolether such as laureth-n, ceteareth-n, steareth-n, oleth-n. Fatty acid polyglycolether such as PEG-n stearate, PEG-n oleate, PEG-n cocoate. Monoglycerides and polyol esters. C12-C22 fatty acid mono- and di-esters of addition products of from 1 to 30 mol of ethylene oxide with polyols. Fatty acid and polyglycerol ester such as monostearate glycerol, diisostearoyl polyglyceryl-3-diisostearates, polyglyceryl-3-diisostearates, triglyceryl diisostearates, polyglyceryl-2-sesquiisostearates or polyglyceryl dimerates. Mixtures of compounds from a plurality of those substance classes are also suitable. Fatty acid polygly-colesters such as monostearate diethylene glycol, fatty acid and polyethylene glycol esters, fatty acid and saccharose esters such as sucro esters, glycerol and saccharose esters such as sucro glycerides. Sorbitol and sorbitan, sorbitan mono- and di-esters of saturated and unsaturated fatty acids having from 6 to 22 carbon atoms and ethylene oxide addition products. Polysorbate-n series, sorbitan esters such as sesquiisostearate, sorbitan, PEG-(6)-isostearate sorbitan, PEG-(10)-sorbitan laurate, PEG-17- dioleate sorbitan. Glucose derivatives, C8-C22 alkyl-mono and oligo-glycosides and ethoxylated analogues with glucose being preferred as the sugar component. O/W emulsifiers such as methyl gluceth-20 sesquistearate, sorbitan stearate/sucrose cocoate, methyl glucose sesquistearate, cetearyl alcohol/cetearyl glucoside. W/O emulsifiers such as methyl glucose dioleate/ methyl glucose isostearate. Sulfates and sulfonated derivatives, dialkylsulfosuccinates, dioctyl succinate, alkyl lauryl sulfonate, linear sulfonated parafins, sulfonated tetraproplyne sulfonate, sodium lauryl sulfates, amonium and ethanolamine lauryl sulfates, lauyl ether sulfates, sodium laureth sulfates, sulfosuccinates, aceyl isothionates, alkanolamide sulfates, taurines, methyl taurines, imidazole sulfates. Amine derivatives, amine salts, ethoxylated amines, oxide amine with chains containing an heterocycle such as alkyl imidazolines, pyridine derivatives, isoquinoteines, cetyl pyridinium chlorure, cetyl pyridinium bromide, quaternary ammonium such as cetyltrimethylbroide amonium broide (CTBA), stearylalkonium. Amide derivatives, alkanolamides such as acylamide DEA, ethoxylated amides such as PEG-n acylamide, oxydeamide. Polysiloxane/polyalkyl/polyether copolymers and derivatives, dimethicone, copolyols, silicone polyethylene oxide copolymer, silicone glycol copolymer. Propoxylated or POE-n ethers (Meroxapols), Polaxamers or poly(oxyethylene)m-block-poly(oxypropylene)n-block(oxyethylene). Zwitterionic surfactants that carry at least one quaternary ammonium group and at least one carboxylate and/or sulfonate group in the molecule. Zwitterionic surfactants that are especially suitable are betaines, such as N alkyl-N,N dimethylammonium glycinates, cocoalkyldimethylammonium glycinate, N acylaminopropyl N,N dimethylammonium glycinates, cocoacylaminopropyldimethylammonium glycinate and 2 alkyl-3-carboxymethyl-3-hydroxyethylimidazolines each having from 8 to 18 carbon atoms in the alkyl or acyl group and also cocoacylaminoethylhydroxyethylcarboxymethylglycinate, N-alkylbetaine, N-alkylaminobetaines. Alkylimidazolines, alkylopeptides, lipoaminoacides, self emulsifying bases and the compounds as described in K.F.DePolo, A short textbook of cosmetology, Chapter 8, Table 8-7, p250-251, which are incorporated herein by reference.
Non ionic emulsifiers such as PEG-6 beeswax (and) PEG-6 stearate (and) polyglyceryl-2 isostearate [Apifac], glyceryl stearate (and) PEG-100 stearate. [Arlacel 165], PEG-5 glyceryl stearate [arlatone 983 S], sorbitan oleate (and) polyglyceryl-3 ricinoleate.[Arlacel 1689], sorbitan stearate and sucrose cocoate [arlatone 2121], glyceryl stearate and laureth-23 [Cerasynth 945], cetearyl alcohol and ceteth-20 [Cetomacrogol Wax], cetearyl alcohol and colysorbate 60 and PEG-150 and stearate-20[Polawax GP 200, Polawax NF], cetearyl alcohol and cetearyl polyglucoside [Emulgade PL 1618], cetearyl alcohol and ceteareth-20 [Emulgade 1000NI, Cosmowax], cetearyl alcohol and PEG-40 castor oil [Emulgade F Special], cetearyl alcohol and PEG-40 castor oil and sodium cetearyl sulfate [Emulgade F], stearyl alcohol and steareth-7 and steareth-10 [Emulgator E 2155], cetearyl alcohol and steareth-7 and steareth-10 [Emulsifying wax U.S.N.F], glyceryl stearate and PEG-75 stearate [Gelot 64], propylene glycol ceteth-3 acetate .[Hetester PCS], propylene glycol isoceth-3 acetate [Hetester PHA], cetearyl alcohol and ceteth-12 and oleth-12 [Lanbritol Wax N 21], PEG -6 stearate and PEG-32 stearate [Tefose 1500], PEG-6 stearate and ceteth-20 and steareth-20 [Tefose 2000], PEG-6 stearate and ceteth-20 and glyceryl stearate and steareth-20 [Tefose 2561], glyceryl stearate and ceteareth-20 [Teginacid H, C, X].
Anionic emulsifiers such as PEG-2 stearate SE, glyceryl stearate SE [Monelgine, Cutina KD], propylene glycol stearate [Tegin P], cetearyl Alcohol and Sodium cetearyl sulfate [Lanette N, Cutina LE, Crodacol GP], cetearyl alcohol and sodium lauryl sulfate [Lanette W], trilaneth-4 phopshate and glycol stearate and PEG-2 stearate [Sedefos 75], glyceryl stearate and sodium lauryl Sulfate [Teginacid Special]. Cationic acid bases such as cetearyl alcohol and cetrimonium bromide.
The emulsifiers may be used in an amount of, for example, from 1 to 30 % by weight, especially from 4 to 20 % by weight and preferably from 5 to 10 % by weight, based on the total weight of the composition.
When formulated in O/W emulsions, the preferably amount of such emulsifier system could represent 5% to 20% of the oil phase.

### Adjuvants and additives

The cosmetic/pharmaceutical preparations, for example creams, gels, lotions, alcoholic and aqueous/alcoholic solutions, emulsions, wax/fat compositions, stick preparations, powders or ointments, may in addition contain, as further adjuvants and additives, mild surfactants, super-fatting agents, consistency regulators, thickeners, polymers, stabilisers, biogenic active ingredients, deodorising active ingredients, anti-dandruff agents, film formers, swelling agents, further UV light-protective factors, antioxidants, hydrotropic agents, preservatives, insect repellents, self-tanning agents, solubilisers, perfume oils, colourants, bacteria-inhibiting agents and the like.

### Super-fatting agents

Substances suitable for use as super-fatting agents are, for example, lanolin and lecithin and also polyethoxylated or acrylated lanolin and lecithin derivatives, polyol fatty acid esters, monoglycerides and fatty acid alkanolamides, the latter simultaneously acting as foam stabilisers.

### Surfactants

Examples of suitable mild surfactants, that is to say surfactants especially well tolerated by the skin, include fatty alcohol polyglycol ether sulfates, monoglyceride sulfates, mono- and/or di-alkyl sulfosuccinates, fatty acid isethionates, fatty acid sarcosinates, fatty acid taurides, fatty acid glutamates, a-olefin sulfonates, ethercarboxylic acids, alkyl oligoglucosides, fatty acid glucamides, alkylamidobetaines and/or protein fatty acid condensation products, the latter preferably being based on wheat proteins.

### Consistency regulators/thickeners and rheology modifiers

Silicium dioxide, magnesium silicates, aluminium silicates, polysaccharides or derivatives thereof for example hyaluronic acid, xanthan gum, guar-guar, agar-agar, alginates, carraghenan, gellan, pectines, or modified cellulose such as hydroxycellulose, hydroxypropylmethylcellulose. In addition polyacrylates or homopolymer of reticulated acrylic acids and polyacrylamides, carbomer (carbopol types 980, 981, 1382, ETD 2001, ETD2020, Ultrez 10) or Salcare range such as Salcare SC80(steareth-10 allyl ether/acrylates copolymer), Salcare SC81 (acrylates copolymer), Salcare SC91 and Salcare AST(sodium acrylates copolymer/PPG-1 trideceth-6), sepigel 305(polyacrylamide/laureth-7), Simulgel NS and Simulgel EG (hydroxyethyl acrylate / sodium acryloyldimethyl taurate copolymer), Stabilen 30 (acrylates / vinyl isodecanoate crosspolymer), Pemulen TR-1 (acrylates / C10-30 alkyl acrylate crosspolymer), Luvigel EM (sodium acrylates copolymer), Aculyn 28 (acrylates/beheneth-25 methacrylate copolymer), etc.

### Polymers

Suitable cationic polymers are, for example, cationic cellulose derivatives, for example a quaternised hydroxymethyl cellulose obtainable under the name Polymer JR 400 from Amerchol, cationic starches, copolymers of diallylammonium salts and acrylamides, quarternised vinylpyrrolidone/vinyl imidazole polymers, for example Luviquat® (BASF), condensation products of polyglycols and amines, quaternised collagen polypeptides, for example lauryldimonium hydroxypropyl hydrolyzed collagen (Lamequat®L/Grünau), quaternised wheat polypeptides, polyethyleneimine, cationic silicone polymers, for example amidomethicones, copolymers of adipic acid and dimethylaminohydroxypropyldiethylenetriamine (Cartaretin/Sandoz), copolymers of acrylic acid with dimethyldiallylammonium chloride (Merquat 550 / Chemviron), polyaminopolyamides, as described, for example, in FR-A-2 252 840, and the crosslinked watersoluble polymers thereof, cationic chitin derivatives, for example of quaternised chitosan, optionally distributed as microcrystals; condensation products of dihaloalkyls, for example dibromobutane, with bisdialkylamines, for example bisdimethylamino-1,3-propane, cationic guar gum, for example Jaguar C-17, Jaguar C-16 from Celanese, quaternised ammonium salt polymers, for example Mirapol A-15, Mirapol AD-1, Mirapol AZ-1 from Miranol. As anionic, zwitterionic, amphoteric and non-ionic polymers there come into consideration, for example, vinyl acetate / crotonic acid copolymers, vinylpyrrolidone / vinyl acrylate copolymers, vinyl acetate / butyl maleate / isobornyl acrylate copolymers, methyl vinyl ether /maleic anhydride copolymers and esters thereof, uncrosslinked polyacrylic acids and polyacrylic acids crosslinked with polyols, acrylamidopropyltrimethylammonium chloride /acrylate copolymers, octyl acrylamide/methyl methacrylatetert butylaminoethyl methacrylate/2-hydroxypropyl methacrylate copolymers, polyvinylpyrrolidone, vinylpyrrolidone/vinyl acetate copolymers, vinylpyrrolidone/dimethylaminoethyl methacrylate/vinyl caprolactam terpolymers and also optionally derivatised cellulose ethers and silicones. Furthermore the polymers as described in EP 1093796 (pages 3-8, paragraphs 17-68) may be used.

### Biogenic active ingredients

Biogenic active ingredients are to be understood as meaning, for example, tocopherol, tocopherol acetate, tocopherol palmitate, ascorbic acid, deoxyribonucleic acid, retinol, bisabolol, allantoin, phytantriol, panthenol, AHA acids, amino acids, ceramides, pseudoceramides, essential oils, plant extracts and vitamin complexes.

### Deodorising active ingredients

As deodorising active ingredients there come into consideration, for example, antiperspirants, for example aluminium chlorohydrates (see J. Soc. Cosm. Chem. 24, 281 (1973)). Under the trade mark Locron® of Hoechst AG, Frankfurt (FRG), there is available comercially, for example, an aluminium chlorohydrate corresponding to formula Al₂(OH)5Cl x 2.5 H₂O, the use of which is especially preferred (see J. Pharm. Pharmacol. 26, 531 (1975)). Besides the chlorohydrates, it is also possible to use aluminium hydroxyacetates and acidic aluminium/zirconium salts. Esterase inhibitors may be added as further deodorising active ingredients. Such inhibitors are preferably trialkyl citrates, such as trimethyl citrate, tripropyl citrate, triisopropyl citrate, tributyl citrate and especially triethyl citrate (Hydagen CAT, Henkel), which inhibit enzyme activity and hence reduce odour formation. Further substances that come into consideration as esterase inhibitors are sterol sulfates or phosphates, for example lanosterol, cholesterol, campesterol, stigmasterol and sitosterol sulfate or phosphate, dicarboxylic acids and esters thereof, for example glutaric acid, glutaric acid monoethyl ester, glutaric acid diethyl ester, adipic acid, adipic acid monoethyl ester, adipic acid diethyl ester, malonic acid and malonic acid diethyl ester and hydroxycarboxylic acids and esters thereof, for example citric acid, malic acid, tartaric acid or tartaric acid diethyl ester. Antibacterial active ingredients that influence the germ flora and kill or inhibit the growth of sweat-decomposing bacteria can likewise be present in the preparations (especially in stick preparations). Examples include chitosan, phenoxyethanol and chlorhexidine gluconate. 5-chloro-2-(2,4-dichlorophenoxy)-phenol (Triclosan, Irgasan, Ciba Specialty Chemicals Inc.) has also proved especially effective.

### Anti-dandruff agents

As anti-dandruff agents there may be used, for example, climbazole, octopirox and zinc pyrithione. Customary film formers include, for example, chitosan, microcrystalline chitosan, quaternised chitosan, polyvinylpyrrolidone, vinylpyrrolidone/vinyl acetate copolymers, polymers of quaternary cellulose derivatives containing a high proportion of acrylic acid, collagen, hyaluronic acid and salts thereof and similar compounds.

### Antioxidants

In addition to the primary light-protective substances it is also possible to use secondary light-protective substances of the antioxidant kind that interrupt the photochemical reaction chain triggered when UV radiation penetrates the skin or hair. Typical examples of such antioxidants are amino acids (e.g. glycine, histidine, tyrosine, tryptophan) and derivatives thereof, imidazoles (e.g. urocanic acid) and derivatives thereof, peptides, such as D,L-carnosine, D-carnosine, L-carnosine and derivatives thereof (e.g. anserine), carotinoids, carotenes, lycopene and derivatives thereof, chlorogenic acid and derivatives thereof, lipoic acid and derivatives thereof (e.g. dihydrolipoic acid), aurothioglycose, propylthiouracil and other thiols (e.g. thioredoxin, glutathione, cysteine, cystine, cystamine and the glycosyl, N-acetyl, methyl, ethyl, propyl, amyl, butyl, lauryl, palmitoyl, oleyl, linoleyl, cholesteryl and glyceryl esters thereof) and also salts thereof, dilauryl thiodipropionate, distearyl thiodipropionate, thiodipropionic acid and derivatives thereof (esters, ethers, peptides, lipids, nucleotides, nucleosides and salts) and also sulfoximine compounds (e.g. buthionine sulfoximines, homocysteine sulfoximine, buthionine sulfones, penta-, hexa-, hepta-thionine sulfoximine), also (metal) chelating agents (e.g. hydroxy fatty acids, palmitic acid phytic acid, lactoferrin), hydroxy acids (e.g. citric acid, lactic acid, malic acid), humic acid, bile acid, bile extracts, bilirubin, biliverdin, EDTA, EDDS, EGTA and derivatives thereof, unsaturated fatty acids and derivatives thereof (e.g. linolenic acid, linoleic acid, oleic acid), folic acid and derivatives thereof, ubiquinone and ubiquinol and derivatives thereof, vitamin C and derivatives (e.g. ascorbyl palmitate, magnesium ascorbyl phosphate, ascorbyl acetate), tocopherols and derivatives (e.g. vitamin E acetate), vitamin A and derivatives (e.g. vitamin A palmitate) and also coniferyl benzoate of benzoin resin, rutinic acid and derivatives thereof, glycosylrutin, ferulic acid, furfurylidene glucitol, carnosine, butyl hydroxytoluene, butyl hydroxyanisole, nordihydroguaiaretic acid, trihydroxybutyrophenone, uric acid and derivatives thereof, mannose and derivatives thereof, superoxide dismutase, N-[3-(3,5-di-tert-butyl-4-hydroxyphenyl)propionyl]sulfanilic acid (and salts thereof, for example the disodium salts), zinc and derivatives thereof (e.g. ZnO, ZnSO4), selenium and derivatives thereof (e.g. selenium methionine), stilbene and derivatives thereof (e.g. stilbene oxide, trans-stilbene oxide) and the derivatives suitable according to the invention (salts, esters, ethers, sugars, nucleotides, nucleosides, peptides and lipids) of those mentioned active ingredients. HALS (="Hindered Amine Light Stabilizers") compounds may also be mentioned.
Further synthetic and natural antioxidants, in particular suitable for the present invention, are listed e.g. in patent WO 0025731:
Structures 1-3 (page 2), structure 4 (page 6), structures 5-6 (page 7) and compounds 7-33 (page 8-14).
The amount of antioxidants present is usually from 0.001 to 30 % by weight, preferably from 0.01 to 3 % by weight, based on the weight of the UV absorbing composite powder of the invention.

### Hydrotropic agents

To improve the flow behaviour it is also possible to employ hydrotropic agents, for example ethoxylated or non ethoxylated mono-alcohols, diols or polyols with a low number of carbon atoms or their ethers (e.g. ethanol, isopropanol, 1,2-dipropanediol, propyleneglycol, glyerin, ethylene glycol, ethylene glycol monoethylether, ethylene glycol monobutylether, propylene glycol monomethylether, propylene glycol monoethylether, propylene glycol monobutylether, diethylene glycol monomethylether; diethylene glycol monoethylether, diethylene glycol monobutylether and similar products). The polyols that come into consideration for that purpose have preferably from 2 to 15 carbon atoms and at least two hydroxy groups. The polyols may also contain further functional groups, especially amino groups, and/or may be modified with nitrogen. Typical examples are as follows: glycerol, alkylene glycols, for example ethylene glycol, diethylene glycol, propylene glycol, butylene glycol, hexylene glycol and also polyethylene glycols having an average molecular weight of from 100 to 1000 Dalton; technical oligoglycerol mixtures having an intrinsic degree of condensation of from 1.5 to 10, for example technical diglycerol mixtures having a diglycerol content of from 40 to 50 % by weight; methylol compounds, such as, especially, trimethylolethane, trimethylolpropane, trimethylolbutane, pentaerythritol and dipentaerythritol; lower alkyl-glucosides, especially those having from 1 to 8 carbon atoms in the alkyl radical, for example methyl and butyl glucoside; sugar alcohols having from 5 to 12 carbon atoms, for example sorbitol or mannitol; sugars having from 5 to 12 carbon atoms, for example glucose or saccharose; amino sugars, for example glucamine; dialcohol amines, such as diethanolamine or 2-amino-1,3-propanediol.

### Preservatives

Suitable preservatives include, for example, Methyl-, Ethyl-, Propyl-, Butyl- parabens, Benzalkonium chloride, 2-Bromo-2-nitro-propane-1,3-diol, Dehydroacetic acid, Diazolidinyl Urea, 2-Dichloro-benzyl alcohol, DMDM hydantoin, Formaldehyde solution, Methyldibromoglutanitrile, Phenoxyethanol, Sodium Hydroxymethylglycinate, Imidazolidinyl Urea, Triclosan and further substance classes, listed in the following reference: K.F.DePolo - A short textbook of cosmetology, Chapter 7, Table 7-2, 7-3, 7-4 and 7-5, p210-219 , which are incorporated herein by reference.

### Bacteria-inhibiting agents

Typical examples of bacteria-inhibiting agents are preservatives that have a specific action against gram-positive bacteria, such as 2,4,4'-trichloro-2'-hydroxydiphenyl ether, chlorhexidine (1,6-di(4-chlorophenyl-biguanido)hexane) or TCC (3,4,4'-trichlorocarbanilide). A large number of aromatic substances and ethereal oils also have antimicrobial properties. Typical examples are the active ingredients eugenol, menthol and thymol in clove oil, mint oil and thyme oil. A natural deodorising agent of interest is the terpene alcohol farnesol (3,7,11-trimethyl-2,6,10-dodecatrien-1-ol), which is present in lime blossom oil. Glycerol monolaurate has also proved to be a bacteriostatic agent. The amount of the additional bacteria-inhibiting agents present is usually from 0.1 to 2 % by weight, based on the solids content of the preparations.

### Perfume oils

There may be mentioned as perfume oils mixtures of natural and/or synthetic aromatic substances. Natural aromatic substances are, for example, extracts from blossom (lilies, lavender, roses, jasmine, neroli, ylang-ylang), from stems and leaves (geranium, patchouli, petitgrain), from fruit (aniseed, coriander, carraway, juniper), from fruit peel (bergamot, lemons, oranges), from roots (mace, angelica, celery, cardamom, costus, iris, calmus), from wood (pinewood, sandalwood, guaiacum wood, cedarwood, rosewood), from herbs and grasses (tarragon, lemon grass, sage, thyme), from needles and twigs (spruce, pine, Scots pine, mountain pine), from resins and balsams (galbanum, elemi, benzoin, myrrh, olibanum, opoponax). Animal raw materials also come into consideration, for example civet and castoreum. Typical synthetic aromatic substances are, for example, products of the ester, ether, aldehyde, ketone, alcohol or hydrocarbon type. Aromatic substance compounds of the ester type are, for example, benzyl acetate, phenoxyethyl isobutyrate, p-tert-butylcyclohexyl acetate, linalyl acetate, dimethylbenzylcarbinyl acetate, phenylethyl acetate, linalyl benzoate, benzyl formate, ethylmethylphenyl glycinate, allylcyclohexyl propionate, styrallyl propionate and benzyl salicylate. The ethers include, for example, benzyl ethyl ether; the aldehydes include, for example, the linear alkanals having from 8 to 18 hydrocarbon atoms, citral, citronellal, citronellyl oxyacetaldehyde, cyclamen aldehyde, hydroxycitronellal, lilial and bourgeonal; the ketones include, for example, the ionones, isomethylionone and methyl cedryl ketone; the alcohols include, for example, anethol, citronellol, eugenol, isoeugenol, geraniol, linalool, phenyl ethyl alcohol and terpinol; and the hydrocarbons include mainly the terpenes and balsams. It is preferable, however, to use mixtures of various aromatic substances that together produce an attractive scent. Ethereal oils of relatively low volatility, which are chiefly used as aroma components, are also suitable as perfume oils, e.g. sage oil, camomile oil, clove oil, melissa oil, oil of cinnamon leaves, lime blossom oil, juniper berry oil, vetiver oil, olibanum oil, galbanum oil, labolanum oil and lavandin oil. Preference is given to the use of bergamot oil, dihydromyrcenol, lilial, lyral, citronellol, phenyl ethyl alcohol, hexyl cinnamaldehyde, geraniol, benzyl acetone, cyclamen aldehyde, linalool, boisambrene forte, ambroxan, indole, hedione, sandelice, lemon oil, tangerine oil, orange oil, allyl amyl glycolate, cyclovertal, lavandin oil, muscatel sage oil, damascone, bourbon geranium oil, cyclohexyl salicylate, vertofix coeur, iso-E-Super, Fixolide NP, evernyl, iraldein gamma, phenylacetic acid, geranyl acetate, benzyl acetate, rose oxide, romillat, irotyl and floramat alone or in admixture with one another.

### Colourants

There may be used as colourants the substances that are suitable and permitted for cosmetic purposes, as compiled, for example, in the publication "Kosmetische Färbemittel" of the Farbstoffkommission der Deutschen Forschungsgemeinschaft, Verlag Chemie, Weinheim, 1984, pages 81 to 106. The colourants are usually used in concentrations of from 0.001 to 0.1 % by weight, based on the total mixture.

### Other adjuvants

It is furthermore possible for the cosmetic preparations to contain, as adjuvants, anti-foams, such as silicones, structurants, such as maleic acid, solubilisers, such as ethylene glycol, propylene glycol, glycerol or diethylene glycol, opacifiers, such as latex, styrene/PVP or styrene/acrylamide copolymers, complexing agents, such as EDTA, NTA, alaninediacetic acid or phosphonic acids, propellants, such as propane/butane mixtures, N₂O, dimethyl ether, CO₂, N₂ or air, so-called coupler and developer components as oxidation dye precursors, reducing agents, such as thioglycolic acid and derivatives thereof, thiolactic acid, cysteamine, thiomalic acid or mercaptoethanesulfonic acid, or oxidising agents, such as hydrogen peroxide, potassium bromate or sodium bromate.
Suitable insect repellents are, for example, N,N-diethyl-m-toluamide, 1,2-pentanediol or insect repellent 3535; suitable self-tanning agents are, for example, dihydroxyacetone and/or erythrulose or dihydroxy acetone and/or dihydroxy acetone precursors as described in WO 01/85124 and/or erythrulose.

### Polymeric beads or hollow spheres as SPF enhancers

The combination of the UV-absorbing composite powder of the invention and a UV-absorber combination, disclosed above, with SPF enhancers, such as non-active ingredients like Styrene/acrylates copolymer, silica beads, spheroidal magnesium silicate, crosslinked Polymethylmethacrylates (PMMA ; Micopearl M305 Seppic), can maximize better the UV protection of the products. Holosphere additives (Sunspheres® ISP, Silica Shells Kobo.) deflect radiation and the effective path length of the photon is therefore increased(EP0893119). Some beads, as mentioned previously, provide a soft feel during spreading. Moreover, the optical activity of such beads, e.g.Micropearl M305, cans modulate skin shine by eliminating reflection phenomena and indirectly may scatter the UV light.

### Cosmetic or pharmaceutical preparations

Cosmetic or pharmaceutical formulations are contained in a wide variety of cosmetic preparations. There come into consideration, for example, especially the following preparations:
- skin-care preparations, e.g. skin emulsions, multi-emulsions or skin oils;
- cosmetic personal care preparations, e.g. facial make-up in the form of day creams or powder creams, face powder (loose or pressed), rouge or cream make-up, eye-care preparations, e.g. eyeshadow preparations, mascara, eye-liner, eye creams or eye-fix creams; lip-care preparations, e.g. lipsticks, lip gloss, lip contour pencils, nail-care preparations, such as nail varnish, nail varnish removers, nail hardeners or cuticle removers;
- light-protective preparations, such as sun milks, lotions, creams or oils, sunblocks or tropicals, pre-tanning preparations or after-sun preparations;
- skin-tanning preparations, e.g. self-tanning creams;
- insect-repellents, e.g. insect-repellent oils, lotions, sprays or sticks;

### Presentation forms of the Cosmetic Composition

The final formulations listed may exist in a wide variety of presentation forms, for example:
- in the form of liquid preparations as a W/O, O/W, O/W/O, W/O/W or PIT emulsion and all kinds of microemulsions,
- in the form of a gel,
- in the form of an oil, a cream, milk or lotion,
- in the form of a powder, a lacquer, a tablet or make-up,
- in the form of a stick,
- in the form of a spray (spray with propellent gas or pump-action spray) or an aerosol,
- in the form of a foam, or
- in the form of a paste.

Of special importance as cosmetic preparations for the skin are light-protective preparations, such as sun milks, lotions, creams, oils, sunblocks or tropicals, pretanning preparations or after-sun preparations, also skin-tanning preparations, for example self-tanning creams. Of particular interest are sun protection creams, sun protection lotions, sun protection milk and sun protection preparations in the form of a spray or sun-protection foams.

For example, especially the following cosmetic formulations may be used:
a1) spontaneously emulsifying stock formulation, consisting of the UV absorber according to the invention, PEG-6-C10oxoalcohol and sorbitan sesquioleate, to which water and any desired quaternary ammonium compound, for example 4 % minkamidopropyl dimethyl-2-hydroxyethylammonium chloride or Quaternium 80 is added;
a2) spontaneously emulsifying stock formulation consisting of the UV absorber according to the invention, tributyl citrate and PEG-20-sorbitan monooleate, to which water and any desired quaternary ammonium compound, for example 4 % minkamidopropyl dimethyl-2-hydroxyethylammonium chloride or Quaternium 80 is added;
b) quat-doped solutions of the UV absorber according to the invention in butyl triglycol and tributyl citrate;
c) mixtures or solutions of the UV absorber according to the invention with n alkylpyrrolidone.

Other typical ingredients in such formulations are preservatives, bactericides and bacteriostatic agents, perfumes, dyes, pigments, thickening agents, moisturizing agents, humectants, fats, oils, waxes or other typical ingredients of cosmetic and personal care formulations such as alcohols, poly-alcohols, polymers, electrolytes, organic solvents, silicon derivatives, emollients, emulsifiers or emulsifying surfactants, surfactants, dispersing agents, antioxidants, anti-irritants and anti-inflammatory agents etc.

The instant invention will be further illustrated with reference to the following examples. However, these examples are intended to illustrate the invention and are not to be construed to limit the scope of the invention. That is, the type and ingredient of the cosmetic composition covered by the instant invention will not be limited to those disclosed in the following examples and the cosmetic composition may comprise any ingredient commonly added or used for manufacturing cosmetic products with no limitation.

### Example 1

Purified water 500g was put in a 1 L-beaker and the core particles, spherical silica having an average size of 3µm (SUNSPHERE®, Asahi Glass SI-Tech. Co., Ltd.) 90g was added thereto with stirring by a dispersing mixer. A commercially available aqueous dispersion of the compound of formula (1) (MBBT) containing 50% MBBT in micronized form with an average particle size < 200 nm (TINOSORB® M, Ciba Specialty Chemicals, Basel, Switzerland) was used. An aqueous dispersion containing 10% TINOSORB® M 100g was added to the beaker and the mixture was stirred well. Then, 5% aqueous aluminum sulfate 100g was added to the mixture and further stirred. After that, pH of the mixture was adjusted to neutral and the mixture was further stirred.

After that, the mixture was centrifuged to dehydrate and the obtained particles were washed with ethanol to remove water soluble ingredients in TINOSORB® M other than the water insoluble UV absorber(MBBT). This step was repeated twice and then, the particles were dried at 120°C for 10 hours to give 100g of composite particles consisting of the core particles and about 5wt% MBBT adsorbed to the surface of the core particles.

### Example 2

Purified water 500g was put in a 1 L-beaker and the core particles, spherical silica with an average size of 3µm (SUNSPHERE®, Asahi Glass Sl-Tech. Co., Ltd.) 89.5g were added thereto with stirring by a dispersing mixer. An aqueous dispersion containing 14% TINOSORB® M 100g was added to the beaker and the mixture was stirred well. Then, 3.5% aqueous aluminum sulfate 100g was added to the mixture and further stirred. After that, pH of the mixture was adjusted to neutral and the mixture was further stirred.
After that, the mixture was treated according to the same manner as Example 1 to give 100g of composite particles consisting of the core particles and about 7wt% MBBT adsorbed to the surface of the core particles.

### Example 3

Purified water 500g was put in a 1 L-beaker and the core particles, spherical silica with an average size of 3µm (SUNSPHERE®, Asahi Glass SI-Tech. Co., Ltd.) 85g was added thereto with stirring by a dispersing mixer. An aqueous dispersion containing 20% TINOSORB® M 100g was added to the beaker and the mixture was stirred well. Then, 5% aqueous aluminum sulfate 100g was added to the mixture and further stirred. After that, pH of the mixture was adjusted to neutral and the mixture was further stirred.
The mixture was treated according to the same manner as Example 1 to give 100g of composite particles consisting of the core particles and about 10wt% MBBT adsorbed to the surface of the core particles.

### Example 4

Purified water 500g was put in a 1 L-beaker and the core particles, spherical silica with an average size of 3µm (SUNSPHERE®, Asahi Glass SI-Tech. Co., Ltd.) 70g was added thereto with stirring by a dispersing mixer. An aqueous dispersion containing 40% TINOSORB® M 100g was added to the beaker and the mixture was stirred well. Then, 10% aqueous aluminum sulfate 100g was added to the mixture and further stirred. After that, pH of the mixture was adjusted to neutral and the mixture was further stirred.
After that, the mixture was treated according to the same manner as Example 1 to give 100g of composite powder consisting of the core particles and about 20wt% MBBT adsorbed to the surface of the core particles.

### Comparative Example 1

Purified water 500g was put in a 1 L-beaker and the core particles, spherical silica with an average size of 3µm (SUNSPHERE®, Asahi Glass SI-Tech. Co., Ltd.) 94g was added thereto with stirring by a dispersing mixer. An aqueous dispersion containing 10% TINOSORB® M 100g was added to the beaker and the mixture was stirred well. Then, 1% aqueous aluminum sulfate 100g was added to the mixture and further stirred. After that, pH of the mixture was adjusted to neutral and the mixture was further stirred.

The mixture was centrifuged to dehydrate. Upon the dehydration, white liquid that seemed to contain MBBT was removed. Then, the particles were washed with ethanol and the filtrate was also white liquid. The amount of the resulting particles was 96g.

### Comparative Example 2

Purified water 500g was put in a 1 L-beaker and the core particles, spherical silica with an average size of 3µm (SUNSPHERE®, Asahi Glass SI-Tech. Co., Ltd.) 98g was added thereto with stirring by a dispersing mixer. 2% aqueous aluminum sulfate 100g was added thereto and the mixture was further stirred. After that, pH of the mixture was adjusted to neutral and the mixture was further stirred. Then, the mixture was centrifuged to dehydrate and the particles were dried at 120°C for 10 hours to give 100g of composite powder.

### Comparative Example 3

Purified water 500g was put in a 1 L-beaker and the core particles, spherical silica with an average size of 3µm (SUNSPHERE®, Asahi Glass Sl-Tech. Co., Ltd.) 40g was added thereto with stirring by a dispersing mixer. An aqueous dispersion containing 80% TINOSORB® M 200g was added to the beaker and the mixture was stirred well. Then, 20% aqueous aluminum sulfate 100g was added to the mixture and further stirred. After that, pH of the mixture was adjusted to neutral and the mixture was further stirred.
The mixture was treated according to the same manner as Example 1 to give 100g of composite particles.

### Test Example 1

### UV-Protecting Effect I (UV-absorbing property)

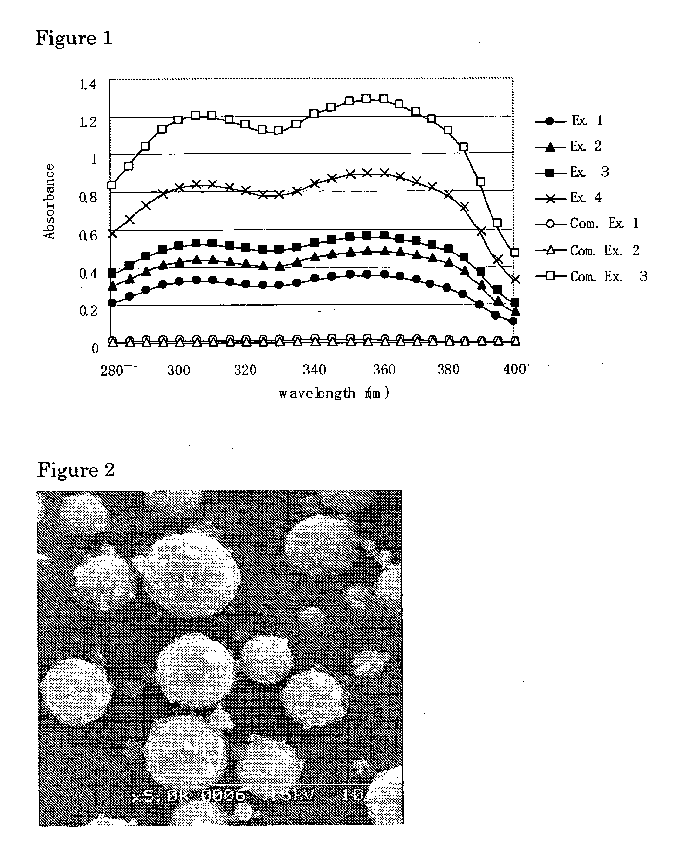

Each of the composite powders comprising the composite powder particles obtained in Examples 1-4 and Comparative Examples 1-3 was weighted and mixed with trioctanoine so that the weight ratio of the composite powder:trioctanoine was 3:7. The mixture was stirred with dispersing mixer at 3000rpm for 10 minutes to give dispersion. The absorption spectrum of each dispersion over the wavelength range of 280-400 nm was determined with a spectrophotometer (UV-3010; HITACHI, Ltd.). Results are shown in Figure 1.

### Test Example 2

### UV-absorbing Effect II (in vitro SPF determination)

Each of the composite powders obtained in Examples 1-4 and Comparative Example 1-3 was weighted and mixed with vaseline so that the weight ratio of the composite powder:vaseline was 25:75. The mixture was homogenized in a mortar to give uniform paste. The paste was uniformly applied on Transpore Tape (Sumitomo 3M) in an amount of 2mg/cm² and in vitro SPF was determined with SPF Analyzer UV-1000S (Labsphere). Result are shown in Table 1 below.

### Test Example 3

### Texture Evaluation

The texture of each composite powder of Examples 1-4 and Comparative Examples 1-3 in view of the touch of the powder is smooth, squeaky or rough was evaluated by 5 panelists according to the five scores shown below:

Five absolute evaluation
1: Bad and uncomfortable texture
2: Slightly bad and uncomfortable texture
3: Normal (comparative to the untreated core particles)
4: Slightly good and comfortable texture
5: Good and comfortable texture

The average values were taken and shown in Table 1 using the following marks:
++: 4 or above
+: 3 or above and less than 4
-: 2 or above and less than 3
--: less than 2

### Results of Test Examples 2 and 3

**[Table 1]**

| | Test Ex. 2 | Test Ex. 3 |
|---|---|---|
| Example 1 | 2.59 | ++ |
| Example 2 | 3.32 | ++ |
| Example 3 | 4.32 | ++ |
| Example 4 | 7.42 | + |
| Com. Ex. 1 | 1.01 | ++ |
| Com. Ex. 2 | 0 | ++ |
| Com. Ex. 3 | 10.42 | -- |

According to figure 1 showing the result of test example 1, the UV absorbance of Examples 1-4 and Comparative Example 3 were increased with the amount of the MBBT adsorbed to the surface of the core particles. Although the powder of comparative Example 1 was manufactured using the same amount of MBBT as that of Example 1, the powder of Comparative Example 1 exhibited less UV absorbance than Example 1. This fact exemplifies that the metal salt like aluminum sulfate is essential for manufacturing the composite powder of the invention. In the Test Example 2, similar result as the Test Example 1 was obtained.

According to the result of Test Example 3, the composite powder of comparative example 3 comprising 40wt% of MBBT provided uncomfortable and rough feeling besides its high UV absorbing property.

### Example 5

Purified water 500g was put in a 1 L-beaker and core particle, mica with an average size of 17µm 70g was added thereto with stirring by a dispersing mixer. An aqueous dispersion containing 40% TINOSORB® M 100g was added to the beaker and the mixture was stirred well. Then, 10% aqueous aluminum sulfate 100g was added to the mixture and further stirred. After that, pH of the mixture was adjusted to neutral and the mixture was further stirred.

The mixture was treated according to the same manner as Example 1 to give 100g of composite powder consisting of the core particles and about 20wt% MBBT adsorbed to the surface of the core particles.

### Examples 6 and 7, Comparative Examples 4-6

### Two-way cake foundation

The composite powder obtained in Example 3 or 4 was used to give two-way cake foundation with the ingredients shown in Table 2 below. The evaluation according to Test Examples 2 and 3 were conducted and results are shown in Table 2.

### Preparation:

Powder ingredients (1)-(11) of table 2 were mixed and pulverized by means of a Henschel mixer. Oil ingredients (12)-(16) of table 2 were mixed in a separate container and added to the powder ingredients. The mixture was kneaded uniformly, then pulverized with an atomizer and the obtained powder was put in an aluminum dish and pressed to give two way cake foundation.

**[Table 2]**

| Ingredients of the two way cake foundation | | | | | | | |
|---|---|---|---|---|---|---|---|
| Ingredients | | amount(wt%) | | | | | |
| | | Ex. 6 | Ex. 7 | Comp. Ex. 4 | Comp. Ex. 5 | Comp. Ex. 6 | Comp. Ex. 7 |
| (1) | methicone treated talc | balance | balance | balance | balance | balance | balance |
| (2) | methicone treated titanium dioxide | 6 | 6 | 6 | 6 | 6 | 6 |
| (3) | methicone treated mica | 20 | 20 | 20 | 20 | 20 | 20 |
| (4) | methione treated sericite | 36 | 36 | 36 | 36 | 36 | 36 |
| (5) | Composite powder Ex. 3 | 10 | | | | | |
| (6) | Composite Powder Ex. 4 | | 10 | | | | |
| (7) | Micronized titanium dioxide | | | 3 | 5 | | 3 |
| (8) | Micronized zinc oxide | | | | | 5 | 5 |
| (9) | methicone treated yellow iron oxide | 1 | 1 | 1 | 1 | 1 | 1 |
| (10) | methicone treated red iron oxide | 0.5 | 0.5 | 0.5 | 0.5 | 0.5 | 0.5 |
| (11) | methicone treated black iron oxide | 0.1 | 0.1 | 0.1 | 0.1 | 0.1 | 0.1 |
| (12) | Dimethicone 1000cs | 6 | 6 | 6 | 6 | 6 | 6 |
| (13) | Octyl methoxy cinnamate | 3 | 3 | 3 | 3 | 3 | 3 |
| (14) | squalane | 3 | 3 | 3 | 3 | 3 | 3 |
| (15) | Preservative | 0.2 | 0.2 | 0.2 | 0.2 | 0.2 | 0.2 |
| (16) | antioxidant | 0.1 | 0.1 | 0.1 | 0.1 | 0.1 | 0.1 |
| | total | 100.0 | 100.0 | 100.0 | 100.0 | 100.0 | 100.0 |

**[Table 3]**

| | Ex. 6 | Ex. 7 | Comp. Ex. 4 | Comp. Ex. 5 | Comp. Ex. 6 | Comp. Ex. 7 |
|---|---|---|---|---|---|---|
| in vitro SPF | 20.4 | 25.4 | 15.2 | 20.3 | 18.2 | 28.0 |
| texture | ++ | ++ | ++ | - | ± | - |

As is apparent from table 3 above, the two way cake foundations of Examples 6 and 7 exhibited excellent UV protecting effects. The compositions of comparative examples 5-7 also exhibited an excellent UV-protecting effect but the texture was bad and provided bluish color upon applied to the face. According to the results, the two way foundations of the examples 6 and 7 are excellent in both of UV-protecting property and texture.

### Example 8

### Pressed Powder

**[Table 4]**

| Ingredients | | amount (wt%) |
|---|---|---|
| (1) | talc | balance |
| (2) | polymethyl methacrylate (7µm)* | 10 |
| (3) | sericite | 30 |
| (4) | scaly-shaped silica ** | 3 |
| (5) | composite powder Ex.4 | 10 |
| (6) | preservative | suitable amount |
| (7) | colorant | suitable amount |
| (8) | octyl methoxy cinnamate | 3 |
| (9) | squalane | 2 |
| (10) | Preservative | suitable amount |
| (11) | antioxidant | suitable amount |
| (12) | perfume | suitable amount |

| | | |
|---|---|---|
| *Matsumoto Microsphere®M-100 (Matsumoto Yushi Seiyaku Co., Ltd) **SUNLOVELY®C (Asahi Glass SI-Tech. Co.,Ltd.) | | |

### Preparation:

Powder ingredients (1)-(7) of table 4 were mixed and pulverized, and put in a Henschel mixer. The oil ingredients (8)-(11) of table 4 were added into the mixer and kneaded to give an uniform mixture. The mixture was then pulverized with an atomizer, put in an aluminum dish and pressed to give pressed powder.

### Evaluation:

The obtained pressed powder had a smooth and comfortable texture and an in-vitro SPF of 17.

### Example 9

### Oil Based Cake Foundation

**[Table 5]**

| Ingredients | | amount (wt%) |
|---|---|---|
| (1) | methicone treated talc | 5.3 |
| (2) | methicone treated titanium dioxide | 8 |
| (3) | composite powder Ex. 5 | 15 |
| (4) | methicone treated sericite | 28.2 |
| (5) | methicone treated iron oxide red | 0.5 |
| (6) | methicone treated iron oxide yellow | 1.8 |
| (7) | methicone treated iron oxide black | 0.2 |
| (8) | candelilla wax | 1 |
| (9) | carnauba wax | 1 |
| (10) | ceresin | 1.5 |
| (11) | cyclopentasiloxane | 14 |
| (12) | isononyl isononanate | balance |
| (13) | Polyglyceryl-2 diisostearate | 2 |
| (14) | dextrin palmitate* | 1 |
| (15) | octyl methoxy cinnamate | 3 |
| (16) | Preservative | suitable amount |
| (17) | antioxidant | suitable amount |

| | | |
|---|---|---|
| *: Rheopearl^{™} KL(Chiba Seifun Co.,Ltd.) | | |

### Preparation:

Powder ingredients (1)-(7) of table 5 were mixed and pulverized in a Henschel mixer. Oil ingredients (8)-(17) of table 5 were mixed and heated to melt and dissolved. The powder ingredients were added to the oil ingredients, the mixture was dispersed uniformly and put in a container, gradually cooled to the room temperature to give oil based cake foundation.
The obtained oil based cake foundation had a smooth and comfortable texture and an in-vitro SPF of 20.

### Example 10

### Stick Type Foundation

**[Table 6]**

| Ingredients | | amount (wt%) |
|---|---|---|
| (1) | dimethicone | 18 |
| (2) | cyclopentasiloxane | 30 |
| (3) | octyl methoxy cinnamate | 5 |
| (4) | diisostearyl malate | 4 |
| (5) | candelilla wax | 6 |
| (6) | hydrogenated jojoba wax | 4 |
| (7) | cetyl dimethicone copolyol | 2 |
| (8) | sorbitan sesquiisostearate | 0.5 |
| (9) | antioxidant | suitable amount |
| (10) | Preservative | suitable amount |
| (11) | perfume | suitable amount |
| (12) | methicone treated colorant | 0.5 |
| (13) | methicone treated titanium dioxide | 3.5 |
| (14) | methicone treated talc | 2 |
| (15) | methicone treated mica | 2 |
| (16) | polymethyl methacrylate | 2 |
| (17) | composite powder Ex. 2 | 10 |
| (18) | purified water | balance |
| (19) | sodium citrate | 0.3 |
| (20) | butyleneglycol | 3 |
| (21) | glycerin | 2 |
| (22) | Preservative | suitable amount |

### Preparation:

Powder ingredients (12)-(17) of table 6 were mixed in a Henschel mixer. Oil ingredients (1)-(11) of table 6 were weighted, put in a container and heated to melt. Ingredients (18)-(22) of table 6 were weighted and put in another container and dissolved by heating to give an aqueous solution. The mixture of the powder ingredients was added to the oil ingredients and the resulting mixture was dispersed uniformly. Then the aqueous solution was added thereto, and the mixture was emulsified and defoamed. Thus obtained mixture was put in a mold and cooled gradually to give the desired stick foundation.

### Evaluation:

The obtained stick foundation had a smooth and comfortable texture and an in-vitro SPF of 17.

### Example 11

### W/O Emulsion Foundation

**[Table 7]**

| Ingredients | | amount (wt%) |
|---|---|---|
| (1) | POE-10 dimethicone(HLB=4.5) | 0.80 |
| (2) | Polyglyceryl polyricinoleate | 0.50 |
| (3) | neopentyl glycol dicaprate | 3.00 |
| (4) | squalane | 1.00 |
| (5) | pentaerythrityl tetraethylhexanoate | 2.00 |
| (6) | stearoyl inulin* | 1.00 |
| (7) | octyl methoxy cinnamate | 4.00 |
| (8) | cyclopentasiloxane | 15.40 |
| (9) | Preservative | suitable amount |
| (10) | antioxidant | suitable amount |
| (11) | perfume | suitable amount |
| (12) | silicone treated titanium dioxide | 4.00 |
| (13) | silicone treated talc | 3.68 |
| (14) | composite powder Ex. 3 | 6.00 |
| (15) | silicone treated colorant | 1.00 |
| (16) | purified water | balance |
| (17) | butylene glycol | 6.00 |
| (18) | glycerin | 1.00 |
| (19) | sodium chloride | 1.00 |
| (20) | Preservative | suitable amount |

| | | |
|---|---|---|
| *Rheopearl^{™} ISK(Chiba Seifun Co.,Ltd.) | | |

### Preperation:

Powder ingredients (12)-(15) of table 7 were mixed in a Henschel mixer, added to oil ingredients (1)-(11) of table 7, and the resulting mixture was kneaded uniformly. Ingredients (16)-(20) of table 7 were heated in a separate container to provide an aqueous solution. The mixture wherein powder ingredients were dispersed in the oil ingredients, was added to the aqueous solution and the mixture was emulsified. The emulsion was cooled gradually to the room temperature to give the desired W/O emulsion foundation.

### Evaluation:

The obtained W/O emulsion foundation had a smooth and comfortable texture and an in-vitro SPF of 18.

### Example 12

### Cheek Blush

**[Table 8]**

| | Ingredients | amount (wt%) |
|---|---|---|
| (1) | talc | balance |
| (2) | sericite | 60.9 |
| (3) | micronized titanium dioxide | 3.0 |
| (4) | composite powder of claim 5 | 17.5 |
| (5) | colorant | suitable amount |
| (6) | octyl methoxy cinnamate | 3.0 |
| (7) | octyl palmitate | 5.0 |
| (8) | Preservative | suitable amount |
| (9) | antioxidant | suitable amount |

### Preparation:

Powder ingredients (1)-(5) of table 8 were mixed by a Henschel Mixer and oil ingredients (6)-(9) of table 8 were dissolved by heating. Both mixtures were mixed and pulverized with an atomizer. The resulting powder was put in a dish and pressed to give the desired pressed cheek color blush.

The obtained pressed cheek color blush had a smooth and comfortable texture and an in-vitro SPF of 25.

### Example 13

### Sunscreen Cream

**[Table 9]**

| | Ingredients | amount (wt%) |
|---|---|---|
| (1) | PEG-10 dimeticone | 3 |
| (2) | cyclopentasiloxane | 15.8 |
| (3) | neopentylglycol dicaprate | 5 |
| (4) | squalane | 5 |
| (5) | dextrin palmitate* | 1.5 |
| (6) | octyl methoxy cinnamate | 3 |
| (7) | preservative | suitable amount |
| (8) | antioxidant | suitable amount |
| (9) | Micronized titanium dioxide 58% dispersion** | 20 |
| (10) | composite powder Ex. 3 | 5 |
| (11) | purified water | balance |
| (12) | butyleneglycol | 4 |
| (13) | peservative | suitable amount |
| (14) | sodium chloride | 1 |

| | | |
|---|---|---|
| *:Rheopearl™ ISK(Chiba Seifun Co.,Ltd.) **:Cosmeserve™ WP-58MP(V)(Dainippon Kasei Co., Ltd.) | | |

### Preparation:

Oil ingredients (1)-(8) of table 9 were dissolved by heating and ingredients (9) and (10) of table 9 were added thereto. The mixture was dispersed uniformly by an agitator. Ingredients (11)-(14) of table 9 were put in a separate container and heated to give an aqueous solution. Said aqueous solution was added to the mixture of ingredients (1)-(10) of table 9 and dispersed to give emulsion, the mixture was cooled to room temperature to give sunscreen cream. Thus obtained sunscreen cream was highly transparent and had a smooth and comfortable texture and an in-vitro SPF of 50.

### Example 14

### Lipstick

**[Table 10]**

| | Ingredients | amount (wt%) |
|---|---|---|
| (1) | carnauba wax | 2.00 |
| (2) | candelilla wax | 5.00 |
| (3) | ceresin | 6.00 |
| (4) | microcrystalline wax | 2.00 |
| (5) | cholesteryl hydroxystearate | 1.50 |
| (6) | diisostearyl malate | 12.00 |
| (7) | glyceryl triisostearate | balance |
| (8) | neopentyl glycol dicaprate | 8.15 |
| (9) | octyldodecanol | 12 |
| (10) | pentaerythritol-fatty acid ester | 12 |
| (11) | octyl methoxy cinnamate | 3 |
| (12) | antioxidant | suitable amount |
| (13) | peservative | suitable amount |
| (14) | colorant | suitable amount |
| (15) | composite powder of Example 5 | 5.00 |
| (16) | Pearl Lustre Pigments | 5.00 |

### Preparation:

Oil ingredients (1)-(13) of table 10 were mixed and dissolved to give an uniform solution, ingredients (14)-(16) of table 10 were added thereto and dispersed uniformly. The mixture was defoamed, poured in a mold and cooled. Thus obtained stick was mounted in a suitable container to give lipstick.

The obtained lipstick had a smooth and comfortable texture and an in-vitro SPF of 10.

### Example 15

### Pore Minimizer Makeup Base

**[Table 11]**

| Ingredients | | amount (wt%) |
|---|---|---|
| (1) | octyl methoxy cinnamate | 5 |
| (2) | methylphenylpolysiloxane | 30.15 |
| (3) | stearylalcohol | 9 |
| (4) | dextrin palmitate* | 7.4 |
| (5) | preservative | suitable amount |
| (6) | antioxidant | suitable amount |
| (7) | cyclopenta siloxane | balance |
| (8) | Composite powder Example 4 | 10 |
| (9) | porous silica** | 20 |

| | | |
|---|---|---|
| *: Rheopearl^{™} KL(Chiba Seifun Co.,Ltd.) **:SUNSPHERE® L-51 (Asahi Glass SI-Tech. Co., Ltd.) | | |

### Preparation:

Oil ingredients (1)-(7) of table 11 were dissolved to give uniform solution. Ingredients (8) and (9) of table 11 were added thereto and dispersed uniformly. The mixture was defoamed and poured in a suitable container and cooled gradually to room temperature to give pore minimizing make up base. The make up base had a smooth and comfortable texture and an in-vitro SPF of 20 (in vitro).

### Example 16

### Loose Powder

**[Table 12]**

| Ingredients | | amount (wt%) |
|---|---|---|
| (1) | talc | balance |
| (2) | titanium dioxide | 1 |
| (3) | lauroyl lysine* | 3 |
| (4) | composite powder Ex. 4 | 10 |
| (5) | Micronized titanium dioxide | 5 |
| (6) | Preservative | suitable amount |
| (7) | colorant | suitable amount |
| (8) | squalane | 1 |
| (9) | preservative | suitable amount |
| (10) | antioxidant | suitable amount |
| (11) | perfume | suitable amount |

| | | |
|---|---|---|
| * Amihope®LL(Ajinomoto Co., Inc.) | | |

### Preparation:

Powder ingredients (1)-(7) of table 12 were pulverized and mixed, and put in a Henschel mixer. The oil ingredients (8)-(11) of table 12 were added thereto and the mixture was kneaded uniformly. Thus obtained uniform mixture was pulverized with an atomizer and the obtained powder was put in an aluminum dish and pressed to give pressed loose powder.

The pressed loose powder had a smooth and comfortable texture and an in-vitro SPF of 15.

The contents of the references cited in the instant specification are herein incorporated by reference.

## Claims

1. A composite powder comprising a plurality of composite powder particles, wherein each of the composite powder particles consist of an inorganic or organic core particle having an average particle diameter of 0.5-1 00µm, and a compound of formula (1): 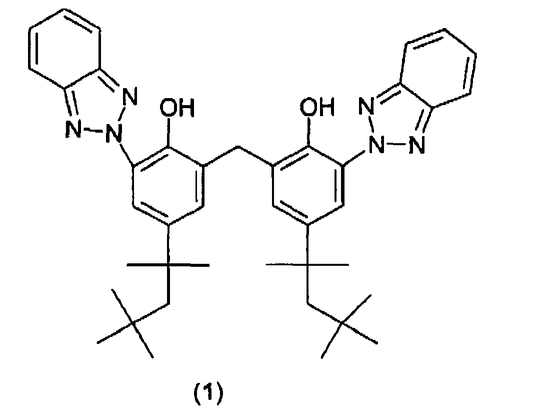 which is adsorbed to the surface of the core particle,
obtainable by
mixing the core particles and the compound of formula (1) in an aqueous solvent under the presence of an aqueous soluble salt of a metal selected from the group consisting of Al, Mg, Ca, Zn, Zr and Ti, whereby the compound of formula (1) adsorbs to the surface of the core particles,
wherein the amount of the aqueous soluble salt of a metal is 0.25 - 2 parts by weight per one part by weight of the compound of formula (1).

2. The composite powder of Claim 1, wherein the compound of formula (1) is in the form of fine particles having an average particle diameter of equal to or less than 200nm.

3. The composite powder of Claim 1 or 2, wherein the amount of the compound of formula (1) is 1-35 wt% based on the total amount of the core particles and the compound of formula (1).

4. A method for manufacturing the composite powder of Claim 1, which comprises the step of:
mixing the core particles and the compound of formula (1) in an aqueous solvent under the presence of an aqueous soluble salt of a metal selected from the group consisting of Al, Mg, Ca, Zn, Zr and Ti, whereby the compound of formula (1) adsorbs to the surface of the core particles,
wherein the amount of the aqueous soluble salt of a metal is 0.25 - 2 parts by weight per one part by weight of the compound of formula (1).

5. The method of Claim 4, wherein the compound of formula (1) is in the form of fine particles having an average particle diameter of equal to or less than 200nm.

6. The method of Claim 4 or 5, wherein the amount of the compound of formula (1) is 1-35 wt% based on the total amount of the core particles and the compound of formula (1).

7. A cosmetic composition comprising a composite powder which comprises a plurality of composite powder particles, wherein the composite powder particle consists of an inorganic or organic core particle having an average particle diameter of 0.5-100µm, and a compound of formula (1): 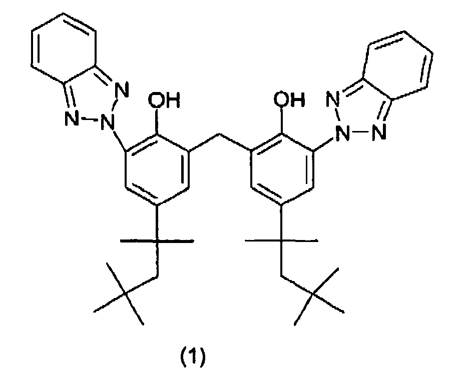 which is adsorbed to the surface of the core particle,
obtainable by
mixing the core particles and the compound of formula (1) in an aqueous solvent under the presence of an aqueous soluble salt of a metal selected from the group consisting of Al, Mg, Ca, Zn, Zr and Ti, whereby the compound of formula (1) adsorbs to the surface of the core particles,
wherein the amount of the aqueous soluble salt of a metal is 0.25 - 2 parts by weight per one part by weight of the compound of formula (1).

8. The cosmetic composition of Claim 7, wherein the compound of formula (1) is in the form of fine particles having an average particle diameter of equal to or less than 200nm.

9. The cosmetic composition of Claim 7 or 8, wherein the amount of the compound of formula (1) is 1-35 wt% based on the total amount of the core particles and the compound of formula (1).

10. The cosmetic composition of any one of Claims 7 to 9 , which comprises one ore morean additional UV absorbers selected from the group consisting of (i) methoxy cinnamate derivatives, (ii) butyl methoxydibenzoylmethane and (iii) mixtures thereof.

11. The cosmetic composition of Claim 10, wherein one or more methoxy cinnamate derivatives are selected from the group consisting of octyl methoxy cinnamate, isoamyl methoxy cinnamate and mixtures thereof.

12. The cosmetic composition of Claim 10 or 11, wherein the additional UV absorber is octyl methoxy cinnamate.

13. The cosmetic composition of any one of Claims 7 to 12, which is selected from the group consisting of powdery foundation, liquid foundation, loose powder, pressed powder, stick foundation, W/O emulsion foundation, cheek blush, sunscreen cream, lipstick and makeup base.

14. Use of a compound of formula (1): 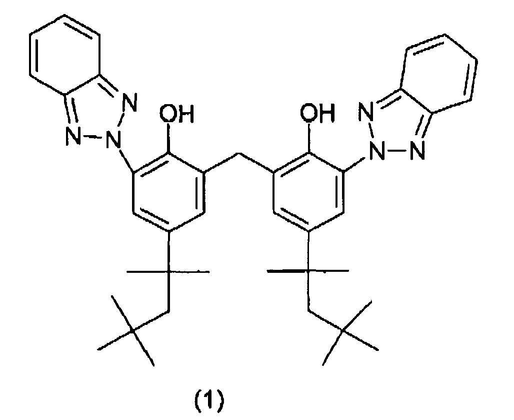 in the manufacture of a composite powder according to any one of Claims 1 to 3.

15. Use of a compound of formula (1): 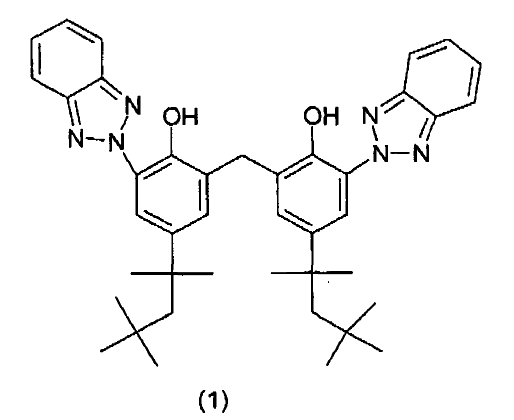 in the manufacture of a cosmetic composition of any one of Claims 7 to 13.

## Patentansprüche

1. Kompositpulver, umfassend eine Vielzahl von Kompositpulverpartikeln, wobei jedes der Kompositpulverpartikel aus einem anorganischen oder organischen Kernpartikel mit einem durchschnittlichen Partikeldurchmesser von 0,5 bis 100µm besteht und einer Verbindung der Formel (1): 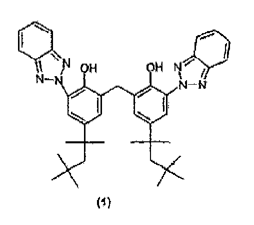 die an die Oberfläche des Kernpartikels adsorbiert ist,
erhältlich durch
Mischen der Kernpartikel und der Verbindung der Formel (1) in einem wässrigen Lösungsmittel in Gegenwart eines wässrigen löslichen Salzes eines Metalls ausgewählt aus der Gruppe bestehend aus Al, Mg, Ca, Zn, Zr und Ti, wodurch die Verbindung der Formel (1) an die Oberfläche der Kernpartikel adsorbiert wird,
wobei die Menge des wässrigen löslichen Salzes eines Metalls 0.25 bis 2 Gewichtsteile pro ein Gewichtsteil der Verbindung der Formel (1) beträgt.

2. Kompositpulver nach Anspruch 1, wobei die Verbindung der Formel (1) in Form feiner Partikel mit einem durchschnittlichen Partikeldurchmesser von gleich oder weniger als 200 nm vorliegt.

3. Kompositpulver nach Anspruch 1 oder 2, wobei die Menge der Verbindung der Formel (1) 1 bis 35 Gew.-% bezogen auf die Gesamtmenge der Kernpartikel und die Verbindung der Formel (1) beträgt.

4. Verfahren zur Herstellung des Kompositpulvers nach Anspruch 1, das den folgenden Schritt umfasst:
Mischen der Kernpartikel und der Verbindung der Formel (1) in einem wässrigen Lösungsmittel in Gegenwart eines wässrigen löslichen Salzes eines Metalls ausgewählt aus der Gruppe bestehend aus Al, Mg, Ca, Zn, Zr und Ti, wodurch die Verbindung der Formel (1) an die Oberfläche der Kernpartikel adsorbiert wird,
wobei die Menge des wässrigen löslichen Salzes eines Metalls 0,25 bis 2 Gewichtsteile pro ein Gewichtsteil der Verbindung der Formel (1) beträgt.

5. Verfahren nach Anspruch 4, wobei die Verbindung der Formel (1) in Form feiner Partikel mit einem durchschnittlichen Partikeldurchmesser von gleich oder weniger als 200 nm vorliegt.

6. Verfahren nach Anspruch 4 oder 5, wobei die Menge der Verbindung der Formel (1) 1 bis 35 Gew.-% bezogen auf die Gesamtmenge der Kernpartikel und die Verbindung der Formel (1) beträgt.

7. Kosmetische Zusammensetzung, umfassend ein Kompositpulver, das eine Vielzahl von Kompositpulverpartikeln umfasst, wobei das Kompositpulverpartikel aus einem anorganischen oder organischen Kernpartikel mit einem durchschnittlichen Partikeldurchmesser von 0,5 bis 100µm besteht, und einer Verbindung der Formel (1): 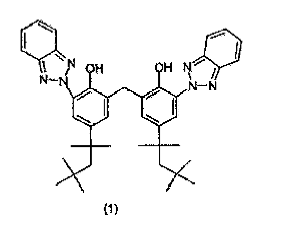 die an die Oberfläche des Kernpartikels adsorbiert ist,
erhältlich durch
Mischen der Kernpartikel und der Verbindung der Formel (1) in einem wässrigen Lösungsmittel in Gegenwart eines wässrigen löslichen Salzes eines Metalls ausgewählt aus der Gruppe bestehend aus Al, Mg, Ca, Zn, Zr und Ti, wodurch die Verbindung der Formel (1) an die Oberfläche der Kernpartikel adsorbiert wird,
wobei die Menge des wässrigen löslichen Salzes eines Metalls 0,25 bis 2 Gewichtsteile pro ein Gewichtsteil der Verbindung der Formel 1 beträgt.

8. Kosmetische Zusammensetzung nach Anspruch 7, wobei die Verbindung der Formel (1) in Form feiner Partikel mit einem durchschnittlichen Partikeldurchmesser von gleich oder weniger als 200 nm vorliegt.

9. Kosmetische Zusammensetzung nach Anspruch 7 oder 8, wobei die Menge der Verbindung der Formel (1) 1 bis 35 Gew.-% bezogen auf die Gesamtmenge der Kernpartikel und die Verbindung der Formel (1) beträgt.

10. Kosmetische Zusammensetzung nach einem der Ansprüche 7 bis 9, die ein oder mehrere zusätzliche UV-Absorptionsmittel umfasst ausgewählt aus der Gruppe bestehend aus (i) Methoxycinnamat-Derivaten, (ii) Butylmethoxydibenzoylmethan und (iii) Gemischen davon.

11. Kosmetische Zusammensetzung nach Anspruch 10, wobei ein oder mehrere Methoxycinnamat-Derivate ausgewählt sind aus der Gruppe bestehend aus Octylmethoxycinnamat, Isoamylmethoxycinnamat und Gemischen davon.

12. Kosmetische Zusammensetzung nach Anspruch 10 oder 11, wobei das zusätzliche UV-Absorptionsmittel Octylmethoxycinnamat ist.

13. Kosmetische Zusammensetzung nach einem der Ansprüche 7 bis 12 ausgewählt aus der Gruppe bestehend aus puderfömiger Grundierung, flüssiger Grundierung, losem Puder, gepresstem Puder, Grundierungsstift, Wasser-in-Öl-Emulsionsgrundierung, Rouge, Sonnencreme, Lippenstift und Make-up-Grundlage.

14. Verwendung einer Verbindung der Formel (1): 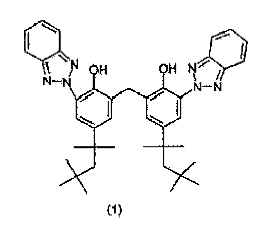 bei der Herstellung eines Kompositpulvers nach einem der Ansprüche 1 bis 3.

15. Verwendung einer Verbindung der Formel (1): 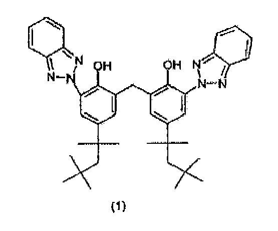 bei der Herstellung einer kosmetischen Zusammensetzung nach einem der Ansprüche 7 bis 13.

## Revendications

1. Poudre composite comprenant une pluralité de particules de poudre composite, chacune des particules de poudre composite étant constituée d'une particule formant noyau inorganique ou organique ayant un diamètre de particule moyen de 0,5 à 100 µm et un composé de formule (1) : 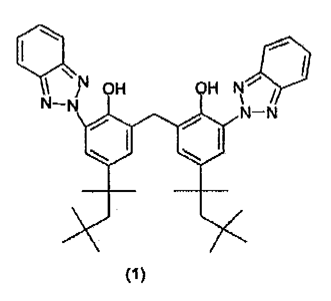 qui est adsorbée à la surface de la particule formant noyau,
pouvant être obtenue par
le mélange des particules formant noyau et du composé de formule (1) dans un solvant aqueux en la présence d'un sel soluble en milieu aqueux d'un métal choisi dans le groupe constitué d'Al, de Mg, de Ca, de Zn, de Zr et de Ti, moyennant quoi le composé de formule (1) s'adsorbe à la surface des particules formant noyau,
dans laquelle la quantité du sel soluble en milieu aqueux d'un métal est de 0,25 à 2 partie(s) en poids pour une partie en poids du composé de formule (1).

2. Poudre composite selon la revendication 1, dans laquelle le composé de formule (1) se présente sous la forme de fines particules ayant un diamètre de particule moyen inférieur ou égal à 200 nm.

3. Poudre composite selon la revendication 1 ou 2, dans laquelle la quantité du composé de formule (1) est de 1 à 35 % en poids sur la base de la quantité totale des particules formant noyau et du composé de formule (1).

4. Procédé de fabrication de la poudre composite selon la revendication 1, qui comprend l'étape suivante :
le mélange des particules formant noyau et du composé de formule (1) dans un solvant aqueux en la présence d'un sel soluble en milieu aqueux d'un métal choisi dans le groupe constitué d'Al, de Mg, de Ca, de Zn, de Zr et de Ti, moyennant quoi le composé de formule (1) s'adsorbe à la surface des particules formant noyau,
dans lequel la quantité du sel soluble aqueux d'un métal est de 0,25 à 2 partie(s) en poids pour une partie en poids du composé de formule (1).

5. Procédé selon la revendication 4, dans lequel le composé de formule (1) se présente sous la forme de fines particules ayant un diamètre de particule moyen inférieur ou égal à 200 nm.

6. Procédé selon la revendication 4 ou 5, dans lequel la quantité du composé de formule (1) est de 1 à 35 % en poids sur la base de la quantité totale des particules formant noyau et du composé de formule (1).

7. Composition cosmétique comprenant une poudre composite qui comprend une pluralité de particules de poudre composite, dans laquelle la particule de poudre composite est constituée d'une particule formant noyau inorganique ou organique ayant un diamètre de particule moyen de 0,5 à 100 µm et un composé de formule (1) : 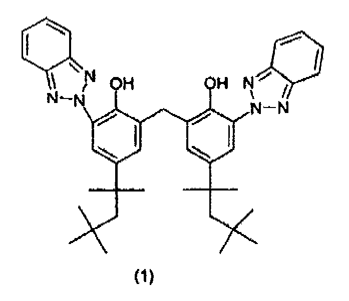 qui est adsorbée à la surface de la particule formant noyau,
pouvant être obtenue par
le mélange des particules formant noyau et du composé de formule (1) dans un solvant aqueux en la présence d'un sel soluble en milieu aqueux d'un métal choisi dans le groupe constitué d'Al, de Mg, de Ca, de Zn, de Zr et de Ti, moyennant quoi le composé de formule (1) s'adsorbe à la surface des particules formant noyau,
dans laquelle la quantité du sel soluble en milieu aqueux d'un métal est de 0,25 à 2 partie(s) en poids pour une partie en poids du composé de formule (1).

8. Composition cosmétique selon la revendication 7, dans laquelle le composé de formule (1) se présente sous la forme de fines particules ayant un diamètre de particule moyen inférieur ou égal de 200 nm.

9. Composition cosmétique selon la revendication 7 ou 8, dans laquelle la quantité du composé de formule (1) est de 1 à 35 % en poids sur la base de la quantité totale des particules formant noyau et du composé de formule (1).

10. Composition cosmétique selon l'une quelconque des revendications 7 à 9, qui comprend un ou plusieurs absorbeurs UV supplémentaires choisis dans le groupe constitué de (i) dérivés de méthoxycinnamate, (ii) butyl méthoxydibenzoylméthane et (iii) mélanges de ceux-ci.

11. Composition cosmétique selon la revendication 10, dans laquelle un ou plusieurs dérivés de méthoxycinnamate sont choisis dans le groupe constitué d' octyl méthoxycinnamate , de méthoxycinnamate d'isoamyle et de mélanges de ceux-ci.

12. Composition cosmétique selon la revendication 10 ou 11, dans laquelle l'absorbeur UV supplémentaire est de l'octyl méthoxycinnamate d.

13. Composition cosmétique selon l'une quelconque des revendications 7 à 12, qui est choisie dans le groupe constitué de fond de teint en poudre, de fond de teint liquide, de poudre libre, de poudre compacte, de fond de teint en bâton, de fond de teint sous forme d'émulsion eau dans huile, de fard à joues, de crème écran solaire, de bâton de rouge à lèvres et de base de maquillage.

14. Utilisation d'un composé de formule (1) : 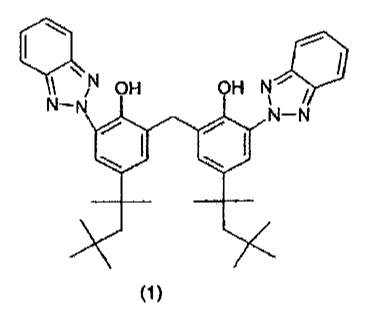 dans la fabrication d'une poudre composite selon l'une quelconque des revendications 1 à 3.

15. Utilisation d'un composé de formule (1) : 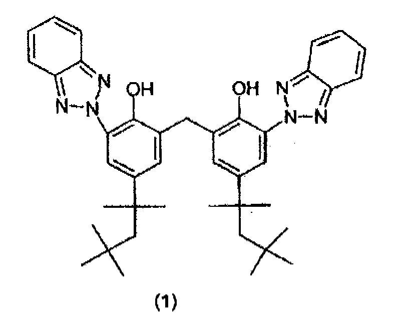 dans la fabrication d'une composition cosmétique selon l'une quelconque des revendications 7 à 13.
